# EUROPEAN PATENT APPLICATION

(11) **EP 4 807 011 A1**
(43) Date of publication of application: **16.09.2026**
(21) Application number: 24889163.2
(22) Date of filing: 08.11.2024
(51) Int. Cl.: C12N 15/86, C07K 14/47, A61K 48/00, A61P 25/00

(54) **ADENO-ASSOCIATED VIRUS VECTOR CAPABLE OF DELIVERING ASTROCYTE-SPECIFIC GENE**

(30) Priority: 08.11.2023 KR 20230153835; 20.11.2023 KR 20230159994
(71) Applicant: Glugenetherapeutics Inc., Daejeon 34109 (KR)
(72) Inventor: JANG, Jae-Hyung, Seoul 07997 (KR); KIM, Joowon, Goyang-si Gyeonggi-do 10546 (KR); LEE, Moon Sue, Seoul 03722 (KR); KANG, Changmin, Seoul 03722 (KR); CHOI, Hongyoung, Seoul 03722 (KR)
(74) Representative: Graf von Stosch Patentanwaltsgesellschaft mbH
(86) International application number: PCT/KR2024/017566
(87) International publication number: WO 2025/100977

(57) **Abstract**

The present invention relates to mutants of adeno-associated virus (AAV) capsid proteins. A recombinant viral vector carrying a mutant of the AAV1 capsid protein is useful for the specific expression of introduced genes in astrocytes at spinal cord injury sites.

## Description

### TECHNICAL FIELD

The present disclosure relates to a variant of the adeno-associated virus (AAV) capsid protein. A recombinant viral vector containing a variant of the AAV1 capsid protein is useful for the expression of transgenes specifically in astrocytes at spinal cord injury sites.

### BACKGROUND ART

For effective gene therapy, the development of a gene delivery technology that allows delivery of therapeutic genes to desired target cells in order to achieve high expression efficiency is the most important.

Among these gene delivery technologies, AAV is a non-pathogenic virus that causes no adverse effects on infected cells and has a low probability of causing mutations in the target cells, making it exceptionally safe as compared to other gene therapy technologies.

AAV (adeno-associated virus) is a non-enveloped, single-stranded DNA virus that can infect both dividing and non-dividing cells. AAV replicates only in the presence of a helper virus and is nonpathogenic to humans. This characteristic makes it a useful method for introducing genes into various cells and a useful vector for gene therapy.

AAV exists as various serotypes, each of which is known to have different host and viral characteristics. Serotype 2 (AAV2) is a serotype that has been widely studied for a long time and can infect a wide range of cells. Serotypes 1 (AAV1), 5 (AAV5), and 6 (AAV6) are serotypes with greater tissue specificity. AAV1 is known to be highly efficient in introducing genes into muscle, liver, respiratory tract, central nervous system, etc., AAV5 into the central nervous system, liver, retina, etc., and AAV6 into the heart, muscle, liver, etc. Although gene delivery characteristics into specific tissues vary depending on the serotype, it is still easy to deliver to other tissues. Therefore, it is essential to develop new AAV vectors that can improve tissue specificity and thus safety and efficiency.

Meanwhile, damage to spinal nerves due to trauma, etc. can lead to paralysis of bodily functions. Spinal cord nerve tissues have a simple structure as compared to the brain, but it is very difficult to regenerate nerves. Pathological phenomena that occur after spinal cord injury can be broadly divided into two stages: primary injury and secondary injury. The primary injury occurs within minutes after injury, with cells at the injury site undergoing necrosis. During this period, cell destruction is so rapid that it is virtually impossible to treat with pharmacological treatment. The secondary injury, which follows the primary injury, progresses slowly over several hours to days. Not only cells at the injury site degenerate, but also surrounding neurons and oligodendrocytes undergo gradual cell death through apoptosis. This cell death continues to progress, centered around the injury site, eventually expanding the damaged area within the spinal cord. In addition, the axons, which are the pathways for nerve signals, and the myelin sheath that helps the axons function degenerate, eventually forming empty spaces like holes, making it impossible for nerve signals to transmit any further, resulting in permanent loss of function.

High-dose methylprednisolone, currently the only treatment approved by the U.S. Food and Drug Administration (FDA), is administered within 8 hours of injury and is intended to reduce acute inflammation, but not to support neurological recovery [Z. Liu et al., High-dose methylprednisolone for acute traumatic spinal cord injury: a meta-analysis. Neurology 93, e841-e850 (2019)]. Therefore, new treatments to promote functional recovery are needed.

Pathologically, traumatic spinal cord injury triggers a series of processes including rapid neuronal cell death, forming a glial scar [M. D. Norenberg, J. Smith, A. Marcillo, The pathology of human spinal cord injury: defining the problems. Journal of Neurotrauma 21, 429-440 (2004)]. This scar is composed primarily of reactive astrocytes, which play a protective role by isolating damaged neurons from healthy tissues in the early stage of injury, but impedes neuronal regeneration [J. E. Burda, M. V. Sofroniew, Reactive gliosis and the multicellular response to CNS damage and disease. Neuron 81, 229-248 (2014)]. Therefore, converting these reactive astrocytes into functional neurons through direct in vivo reprogramming is considered a promising therapeutic strategy.

However, it is difficult to specifically deliver astrocyte reprogramming factors to astrocytes, and when they are delivered to cells other than astrocytes, such as neurons, oligodendrocytes, microglia, etc., normal gene expression can be disrupted, leading to cell death or functional defects. For example, overexpression of these transcription factors in neurons can disrupt gene expression patterns, resulting in abnormal neuronal behavior, loss of function, and even cell death. To ensure safe and effective therapeutic gene delivery or reprogramming to astrocytes surrounding neural injury sites, it is necessary to improve the astrocyte specificity of AAV vectors and minimize conversion in non-target cells.

Therefore, attempts are being made to improve gene transfer efficiency by modifying the AAV capsid protein. However, researches on adeno-associated virus targeting astrocytes at the spinal cord injury site are insufficient.

### DISCLOSURE

### Technical Problem

The inventors of the present disclosure have made consistent efforts to solve the problem described above, and completed the present disclosure by developing a protein variant based on the capsid of a novel adeno-associated virus (AAV) serotype 1 that targets spinal cord astrocytes and has the potential to deliver a gene therapy agent that addresses the fundamental genetic cause of spinal cord injury-related diseases.

### Technical Solution

Therefore, the present disclosure is directed to providing a variant of the AAV1 capsid protein to improve gene transduction efficiency into target tissues or cells by the recombinant AAV and/or improve genetic information expression efficiency.

The present disclosure is also directed to providing a nucleic acid encoding the variant of the AAV1 capsid protein.

The present disclosure is also directed to providing a recombinant AAV1 vector including the nucleic acid encoding the variant of the AAV1 capsid protein.

The present disclosure is also directed to providing a pharmaceutical composition containing the recombinant AAV1 vector.

The present disclosure is also directed to providing a gene delivery vehicle including the recombinant AAV1 vector.

### Advantageous Effects

The present disclosure relates to a recombinant AAV1 capsid variant that specifically targets spinal cord astrocytes and has high-efficiency gene delivery capability. A recombinant viral vector containing a nucleic acid encoding the variant of the AAV1 capsid protein is useful for the expression of transgenes in spinal cord astrocyte target cells, enabling the prevention or treatment of diseases related to spinal cord injury.

### DESCRIPTION OF DRAWINGS

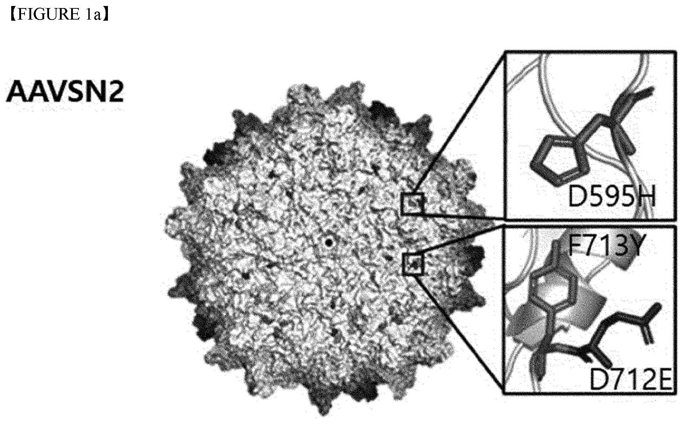

FIG. 1A shows the 3D structure of a recombinant AAV1 variant (AAVSN2 variant). This variant is an AAV1-based variant with three point mutations (D595H, D712E, F713Y), all of which are surface-exposed [red site: 595, blue site: 712, pink site: 713].

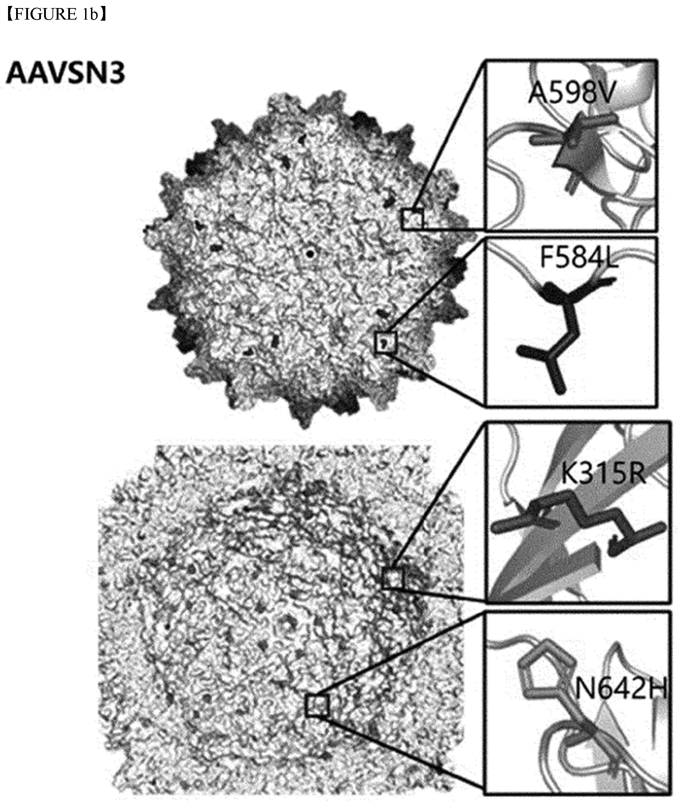

FIG. 1B shows the 3D structure of a recombinant AAV1 variant (AAVSN3 variant). This variant is an AAV1-based variant with four point mutations (K315R, F584L, A598V, and N642H). Of these, the F584L and A598V sites are surface-exposed, while the remaining K315R and N642H sites are internal [red site: 315, blue site: 584, pink site: 598, and orange site: 642].
FIG. 2A shows the cleavage map of a pCMV GFP plasmid.
FIG. 2B shows the cleavage map of a pHelper plasmid.
FIGS. 3A, 3B and 3C show the cleavage maps of a wild-type AAV1 vector, an AAVSN2 variant, and an AAVSN3 variant, respectively.
FIG. 4 shows the cleavage map of a NeuroD1-P2A-GFP plasmid.
FIG. 5 compares the packaging efficiency of a wild-type AAV1 and recombinant AAV1 variants (AAVSN2 and AAVSN3 variants) of the present disclosure using quantitative PCR analysis, based on genomic titers.
FIG. 6 shows the percentage of cells expressing GFP after AAVSN3-CMV-GFP was administered to primary mixed glial cells extracted from the spinal cord of 7-week-old male Sprague-Dawley rats to confirm the astrocyte specificity of a recombinant AAV1 variant (AAVSN3 variant).
FIG. 7A shows a result of directly administering AAVSN2-GFP to injury sites in 7-week-old male Sprague-Dawley rats, extracting astrocytes and then comparing genomic titers with wild-type AAV1-GFP using quantitative PCR 5 days after a contusive spinal cord injury in order to identify the astrocyte transduction ability of a recombinant AAV 1 variant (AAVSN2 variant) at the injury site.
FIG. 7B shows a result of comparing genomic titers at a spinal cord injury site with wild-type AAV1-GFP by conducting experiment using the same method as in FIG. 7A, in order to identify the astrocyte transduction ability of a recombinant AAV1 variant (AAVSN3 variant).
FIG. 7C shows a result of comparing genomic titers with wild-type AAV1 using quantitative PCR at each location in the spinal cord of rats with contusive spinal cord injury in order to identify the transduction ability of a recombinant AAV1 variant (AAVSN3 variant) depending on the distance from the spinal cord injury site.
FIG. 8 shows the ratio of astrocytes expressing the GFP gene via a wild-type AAV1 vector and recombinant AAV1 variants (AAVSN2 and AAVSN3 variants) at the contusive spinal cord injury site of rats, measured using flow cytometry analysis, showing the astrocyte transduction ability of the recombinant AAV1 variants at the lesion site.
FIG. 9 shows the expression level of GFP depending on the distance from the injury site measured by analyzing the images of the GFP gene expression via a wild-type AAV1 vector and recombinant AAV1 variants (AAVSN2 and AAVSN3 variants) at the contusive spinal cord injury site of rats.
FIG. 10 shows a low-magnification image showing the result of local delivery within the nerve injury site confirmed by immunochemical staining at 2 weeks after injection of a recombinant AAV carrying GFP into the spinal cord injury site to confirm the gene delivery and expression ability of a wild-type AAV1 and recombinant AAV1 variants (AAVSN2 and AAVSN3 variants) of the present disclosure in the spinal cord injury site of rats.
FIG. 11A shows the result of confirming the gene delivery ability of a wild-type AAV1 vector and a recombinant AAV1 variant (AAVSN2 variant) in astrocytes near the spinal cord injury site through immunochemical staining and fluorescence imaging at high magnification.
FIG. 11B shows the result of confirming the gene delivery ability of a wild-type AAV1 vector and a recombinant AAV1 variant (AAVSN3 variant) in astrocytes near the spinal cord injury site through immunochemical staining and fluorescence imaging at high magnification.
FIG. 12 shows the result of confirming the gene delivery ability of a wild-type AAV1 vector and recombinant AAV1 variants (AAVSN2 and AAVSN3 variants) in astrocytes near the contusive spinal cord injury site through immunochemical staining and fluorescence imaging at high magnification, and quantifying the delivery ability using the ImageJ program.
FIG. 13 shows the result of confirming the gene delivery ability of a wild-type AAV1 vector and recombinant AAV1 variants (AAVSN2 and AAVSN3 variants) in astrocytes and other cells near the contusive spinal cord injury site [GFAP: astrocytes, Tuj1: nerve cells, Iba1: microglia].
FIG. 14 shows the immune cells in the central nervous system identified by labeling with Iba1 (rabbit anti-Iba1 antibody (1:500 dilution; Wako Pure Chemical Industries)) through immunochemical staining when a wild-type AAV1 vector and recombinant AAV1 variants (AAVSN2 and AAVSN3 variants) carrying the NeuroD1-P2A-GFP gene were administered to the contusive spinal cord injury site.
FIG. 15 shows the results of measuring the viral genome in major organs other than the spinal cord by qPCR and normalizing it to GAPDH when a wild-type AAV1 vector and recombinant AAV1 variants (AAVSN2 and AAVSN3 variants) were administered to the contusive spinal cord injury site.
FIG. 16 shows the change in BBB (Basso, Beattie and Bresnahan) score over time due to recovery of locomotor ability when the NeuroD1-P2A-GFP and CMV-GFP genes were delivered to the contusive spinal cord injury site using a wild-type AAV1 vector and a recombinant AAV1 variant (AAVSN2 variant).
FIG. 17 shows the results comparing the degree of astrocyte reprogramming into neural cells when NeuroD1-P2A-GFP was delivered to the contusive spinal cord injury site by a wild-type AAV1 vector and a recombinant AAV1 variant (AAVSN2 variant).
FIG. 18 shows the change in body weight after introduction of therapeutic genes of recombinant AAV1 variants [(a) AAVSN2 variant; (b) AAVSN3 variant].
FIG. 19 shows the results of blood toxicity tests after introduction of therapeutic genes into recombinant AAV1 variants [(a) AAVSN2 variant; (b) AAVSN3 variant].

### BEST MODE

Hereinafter, the present disclosure is described in more detail.

The present disclosure relates to a variant of an adeno-associated virus serotype 1 (AAV1) capsid protein.

The term "adeno-associated virus" or "AAV" as used in the present disclosure refers to all adeno-associated viruses used in gene therapy, including their derivatives, viral subtypes, and naturally occurring and recombinant forms. Various serotypes of AAV can be used as recombinant gene delivery viruses for transduction into many different cell types. The genomic sequences of various serotypes of AAV, as well as the sequences of the native terminal repeats (TRs), Rep proteins, and capsid subunits, are known in the art. These sequences can be found in the literature or in public databases such as GenBank. For example, see GenBank Accession No. NC_002077 (AAV-1) or AF063497 (AAV-1).

The term "serotype" as used in the present disclosure means a subdivision of AAV that can be identified by serological or DNA sequencing methods and distinguished by its antigenic characteristics.

The term "capsid" used in the present disclosure refers to a protein encoded by the cap gene present in the virus genome, which constitutes the outer shell of the virus. The wild-type AAV genome, or cap gene, encodes three types of capsid proteins (VP1, VP2, and VP3). The wild-type AAV1 capsid protein includes the amino acid sequence represented by SEQ ID NO: 1

In an exemplary embodiment, the present disclosure encompasses a variant of the adeno-associated virus serotype 1 (AAV1) capsid protein, wherein the variant is a variant of the AAV1 capsid protein having one or more mutation selected from D595H, D712E, F713Y, K315R, F584L, A598V, and N642H in the amino acid sequence of SEQ ID NO: 1 of a wild-type AAV1 capsid protein.

The D595H mutation is a substitution of aspartic acid at position 595 in the amino acid sequence of SEQ ID NO: 1 of the wild-type AAV1 capsid protein with histidine.

The D712E mutation is a substitution of aspartic acid at position 712 in the amino acid sequence of SEQ ID NO: 1 of the wild-type AAV1 capsid protein with glutamic acid.

The F713Y mutation is a substitution of phenylalanine at position 713 in the amino acid sequence of SEQ ID NO: 1 of the wild-type AAV1 capsid protein with tyrosine.

The K315R mutation is a substitution of lysine at position 315 in the amino acid sequence of SEQ ID NO: 1 of the wild-type AAV1 capsid protein with arginine.

The F584L mutation is a substitution of phenylalanine at position 584 in the amino acid sequence of SEQ ID NO: 1 of the wild-type AAV1 capsid protein with leucine.

The A598V mutation is a substitution of alanine at position 598 in the amino acid sequence of SEQ ID NO: 1 of the wild-type AAV1 capsid protein with valine.

The N642H mutation is a substitution of asparagine at position 642 in the amino acid sequence of SEQ ID NO: 1 of the wild-type AAV1 capsid protein with histidine.

In an exemplary embodiment, the present disclosure may provide a variant of the adeno-associated virus serotype 1 (AAV1) capsid protein that additionally has one or more mutation selected from T194A, A196S, A197G, T201N, S205A, L129F, A224S, N223S, H272T, S268T, T265S, A263G, S262N and S264T.

The T194A mutation is a substitution of threonine at position 194 in the amino acid sequence of SEQ ID NO: 1 of the wild-type AAV1 capsid protein with alanine,
the A196S mutation is a substitution of alanine at position 196 in the amino acid sequence of SEQ ID NO: 1 of the wild-type AAV1 capsid protein with serine,
the A197G mutation is a substitution of alanine at position 197 in the amino acid sequence of SEQ ID NO: 1 of the wild-type AAV1 capsid protein with glycine,
the T201N mutation is a substitution of threonine at position 201 in the amino acid sequence of SEQ ID NO: 1 of the wild-type AAV1 capsid protein with asparagine,
the S205A mutation is a substitution of serine at position 205 in the amino acid sequence of SEQ ID NO: 1 of the wild-type AAV1 capsid protein with alanine,
the L129F mutation is a substitution of leucine at position 129 in the amino acid sequence of SEQ ID NO: 1 of the wild-type AAV1 capsid protein with phenylalanine,
the A224S mutation is a substitution of alanine at position 224 in the amino acid sequence of SEQ ID NO: 1 of the wild-type AAV1 capsid protein with serine,
the N223S mutation is a substitution of asparagine at position 223 in the amino acid sequence of SEQ ID NO: 1 of the wild-type AAV1 capsid protein with serine,
the H272T mutation is a substitution of histidine at position 272 in the amino acid sequence of SEQ ID NO: 1 of the wild-type AAV1 capsid protein with threonine,
the S268T mutation is a substitution of serine at position 268 in the amino acid sequence of SEQ ID NO: 1 of the wild-type AAV1 capsid protein with threonine,
the T265S mutation is a substitution of threonine at position 265 in the amino acid sequence of SEQ ID NO: 1 of the wild-type AAV1 capsid protein with serine,
the A263G mutation is a substitution of alanine at position 263 in the amino acid sequence of SEQ ID NO: 1 of the wild-type AAV1 capsid protein with glycine,
the S262N mutation is a substitution of serine at position 262 in the amino acid sequence of SEQ ID NO: 1 of the wild-type AAV1 capsid protein with asparagine, and/or
the S264T mutation is a substitution of serine at position 264 in the amino acid sequence of SEQ ID NO: 1 of the wild-type AAV1 capsid protein with threonine.

In an exemplary embodiment, the present disclosure relates to a variant of an adeno-associated virus serotype 1 (AAV1) capsid protein, wherein the variant contains or consists of an amino acid sequence represented by SEQ ID NO: 3 or SEQ ID NO: 5.

In an exemplary embodiment, the variant of the AAV1 capsid protein according to the present disclosure contains or consists of an amino acid sequence represented by SEQ ID NO: 3 or SEQ ID NO: 5 (VP1). VP2 corresponds to the amino acid sequence from T138 to the terminal of SEQ ID NO: 3 or SEQ ID NO: 5, and VP3 corresponds to the amino acid sequence from M203 to the terminal of SEQ ID NO: 3 or SEQ ID NO: 5.

The variant of the AAV1 capsid protein represented by SEQ ID NO: 3 is named SN2, and has mutations of D595H, D712E, and F713Y.

The variant of the AAV1 capsid protein represented SEQ ID NO: 5 is named SN3, and has mutations of K315R, F584L, A598V, and N642H.

In an exemplary embodiment, the present disclosure relates to a variant of an adeno-associated virus serotype 1 (AAV1) capsid protein, wherein the variant has mutations K315R, D595H, D712E, and F713Y.

In an exemplary embodiment, the present disclosure relates to a variant of an adeno-associated virus serotype 1 (AAV1) capsid protein, wherein the variant has mutations F584L, A598V, and N642H.

The term "wild type" used in the present disclosure refers to a form most commonly found in wild populations among species. With regard to mutant types, the wild type refers to a phenotype or individual considered to be the standard. The wild type is also called a normal type as an alias. Meanwhile, in this specification, the term "variant" refers to a protein, virus, cell, individual, etc. in which a gene wherein mutation has occurred shows a phenotypic change. In addition, in this specification, the term "variant" may also refer to the gene itself that has mutated.

In addition, the present disclosure encompasses a nucleic acid encoding the variant of the AAV1 capsid protein.

The nucleic acid of the present disclosure encodes the variant of the AAV1 capsid protein. The nucleic acid of the present disclosure is produced by substituting one base for another base in the base sequence of the nucleic acid encoding the AAV1 capsid protein (cap gene). The nucleic acid of the present disclosure may exist in the form of DNA, but in some cases, it may also exist in the form of RNA or a chimera of DNA and RNA. In addition, the nucleic acid of the present disclosure also includes a complementary nucleic acid (e.g., cDNA). The nucleic acid of the present disclosure may be single-stranded or double-stranded, but is specifically double-stranded.

The wild-type AAV1 cap gene is represented by the base sequence of SEQ ID NO: 2.

The D595H mutation can be encoded by substituting GAT at positions 1783 to 1785 in the base sequence of SEQ ID NO: 2 of the wild-type AAV1 cap gene with CAC or CAT.

The D712E mutation can be encoded by substituting GAT at positions 2134 to 2136 in the base sequence of SEQ ID NO: 2 of the wild-type AAV1 cap gene with GAA or GAG.

The F713Y mutation can be encoded by substituting TTT at positions 2137 to 2139 in the base sequence of SEQ ID NO: 2 of the wild-type AAV1 cap gene with TAT or TAC.

The K315R mutation can be encoded by substituting AAA at positions 943 to 945 in the base sequence of SEQ ID NO: 2 of the wild-type AAV1 cap gene with AGA, AGG, CGA, CGC, CGG, or CGT.

The F584L mutation can be encoded by substituting TTT at positions 1750 to 1752 in the base sequence of SEQ ID NO: 2 of the wild-type AAV1 cap gene with CTA, CTC, CTG, CTT, TTA, or TTG.

The A598V mutation can be encoded by substituting GCT at positions 1792 to 1794 in the base sequence of SEQ ID NO: 2 of the wild-type AAV1 cap gene with GTA, GTC, GTG, or GTT.

The N642H mutation can be encoded by substituting AAC at positions 1924 to 1926 in the base sequence of SEQ ID NO: 2 of the wild-type AAV1 cap gene with CAC or CAT.

The present disclosure relates to a nucleic acid encoding a variant of the AAV1 capsid protein consisting of an amino acid sequence represented by SEQ ID NO: 3 or SEQ ID NO: 5, and a nucleic acid having a base sequence represented by SEQ ID NO: 4 or SEQ ID NO: 6 is exemplified as an exemplary embodiment, although not being specially limited thereto.

The T194A mutation can be encoded by substituting ACC at positions 580 to 582 in the base sequence of SEQ ID NO: 2 of the wild-type AAV1 cap gene with GCA, GCC, GCG, or GCT.

The A196S mutation can be encoded by substituting GCT at positions 586 to 588 in the base sequence of SEQ ID NO: 2 of the wild-type AAV1 cap gene with AGC, AGT, TCA, TCC, TCG, or TCT.

The A197G mutation can be encoded by substituting GCT at positions 599 to 591 in the base sequence of SEQ ID NO: 2 of the wild-type AAV1 cap gene with GGA, GGC, GGG, or GGT.

The T201N mutation can be encoded by substituting ACT at positions 601 to 603 in the base sequence of SEQ ID NO: 2 of the wild-type AAV1 cap gene with AAC or AAT.

The S205A mutation can be encoded by substituting TCA at positions 613 to 615 in the base sequence of SEQ ID NO: 2 of the wild-type AAV1 cap gene with GCA, GCC, GCG, or GCT.

The L129F mutation can be encoded by substituting CTC at positions 385 to 387 in the base sequence of SEQ ID NO: 2 of the wild-type AAV1 cap gene with TTC or TTT.

The A224S mutation can be encoded by substituting GCC at positions 670 to 672 in the base sequence of SEQ ID NO: 2 of the wild-type AAV1 cap gene with AGC, AGT, TCA, TCC, TCG, or TCT.

The N223S mutation can be encoded by substituting AAT at positions 667 to 669 in the base sequence of SEQ ID NO: 2 of the wild-type AAV1 cap gene with AGC, AGT, TCA, TCC, TCG, or TCT.

The H272T mutation can be encoded by substituting CAC at positions 814 to 816 in the base sequence of SEQ ID NO: 2 of the wild-type AAV1 cap gene with ACA, ACC, ACG, or ACT.

The S268T mutation can be encoded by substituting AGC at positions 802 to 804 in the base sequence of SEQ ID NO: 2 of the wild-type AAV1 cap gene with ACA, ACC, ACG, or ACT.

The T265S mutation can be encoded by substituting ACG at positions 793 to 795 in the base sequence of SEQ ID NO: 2 of the wild-type AAV1 cap gene with AGC, AGT, TCA, TCC, TCG, or TCT.

The A263G mutation can be encoded by substituting GCT at positions 787 to 789 in the base sequence of SEQ ID NO: 2 of the wild-type AAV1 cap gene with GGA, GGC, GGG, or GGT.

The S262N mutation can be encoded by substituting AGT at positions 784 to 786 in the base sequence of SEQ ID NO: 2 of the wild-type AAV1 cap gene with AAC or AAT.

The S264T mutation can be encoded by substituting TCA at positions 790 to 792 in the base sequence of SEQ ID NO: 2 of the wild-type AAV1 cap gene with ACA, ACC, ACG, or ACT.

The nucleic acid of the present disclosure may be operably linked to an appropriate regulatory sequence. The regulatory sequence includes a promoter sequence, a polyadenylation signal, a transcription termination sequence, an upstream regulatory domain, an internal ribosome entry site (IRES), an enhancer, etc. The promoter sequence includes an inducible promoter sequence and a constitutive promoter sequence. The regulatory sequence may be native to the AAV from which the capsid protein is derived, or may be foreign. It may also be natural or synthetic. A recombinant DNA capable of expressing a variant of the AAV1 capsid protein containing the nucleic acid of the present disclosure is also included in the present disclosure.

The recombinant DNA is useful for delivering the nucleic acid of the present disclosure to a cell in vitro, ex vivo, and in vivo, and for conferring upon the cell the ability to express a variant of the AAV1 capsid protein. Furthermore, the cell to which the nucleic acid of the present disclosure has been delivered is also useful for producing recombinant AAV particles. The recombinant DNA can be used to deliver or introduce the nucleic acid of the present disclosure into eukaryotic cells, specifically animal cells, more specifically mammalian cells.

In the present disclosure, a recombinant DNA may be produced by including the nucleic acid of the present disclosure in a DNA used as a vector. For example, a plasmid, a phage, a transposon, a cosmid, an episomal DNA, a virus genome, etc. may be used.

For example, a packaging plasmid can be constructed by including a nucleic acid encoding a variant of the AAV1 capsid protein of the present disclosure (cap gene) in a plasmid. The packaging plasmid can additionally include any nucleic acid sequence, such as a nucleic acid encoding the replicase (Rep) protein (rep gene). Specifically, the rep gene can be supplemented with Rep derived from AAV2.

The recombinant DNA including the nucleic acid of the present disclosure can be produced by substituting at least one base in the PLA2 domain coding region with another base, in the nucleic acid sequence of the cap gene loaded in a known packaging plasmid. The packaging plasmid is not particularly limited, but a packaging plasmid loaded with a cap gene, specifically a packaging plasmid loaded with a cap gene and a rep gene, is exemplified. For example, in the present disclosure, a recombinant AAV1 vector represented by SEQ ID NO: 7 or SEQ ID NO: 8, i.e., pxx_AAVSN2 or pxx_AAVSN3, which is a packaging plasmid loaded with a nucleic acid encoding a variant of the AAV1 capsid protein of the present disclosure (cap gene) and a rep gene, was produced.

The method of introducing a base substitution into a nucleic acid can be carried out by known methods and is not particularly limited, but it can be achieved using a commercially available reagent, e.g., Mutagenesis Basal Kit (Takara Bio Inc.) by performing PCR according to the instructions attached to the kit.

Accordingly, the present disclosure provides a recombinant AAV1 vector including a nucleic acid encoding the AAV1 capsid protein variant.

The recombinant AAV vector of the present disclosure is useful for introducing genes into target cells. The gene introduced by the recombinant AAV vector of the present disclosure is strongly expressed in the target cells.

As used herein, the term "AAV vector" refers to any vector that contains or is derived from the components of adeno-associated virus (AAV) and is suitable for infecting mammalian cells, including human cells of any tissue types such as the brain, spinal cord, heart, lung, skeletal muscle, liver, kidney, spleen, or pancreas, whether in vitro or in vivo. The term "AAV vector" may be used to refer to an AAV-type virus particle (or virion) containing at least a nucleic acid molecule encoding a protein of interest.

As used herein, the term "AAV virus," "AAV virus particle," or "rAAV vector particle" refers to a virus particle consisting of at least one AAV capsid protein (of all capsid proteins of wild-type AAV) and a polynucleotide rAAV vector encapsidated into the capsid. If the particle contains a heterogeneous polynucleotide (i.e., a polynucleotide other than the wild-type AAV genome, such as a transgene delivered to a mammalian cell), it is typically referred to as an "rAAV vector particle" or simply an "rAAV vector." Thus, the production of an rAAV particle necessarily involves the production of rAAV, since the vector is contained within the rAAV particle.

The "packaging" refers to a series of intracellular events that cause the assembly of AAV particles and their insertion into the capsid.

The AAV "rep" and "cap" genes refer to polynucleotide sequences encoding the replication of adeno-associated virus insertion of proteins into the capsid. The AAV rep and cap are referred to herein as AAV "packaging genes".

The "helper virus" for AAV refers to a virus that enables AAV (e.g., wild-type AAV) to be replicated and packaged by mammalian cells. Various such helper viruses for AAV are known in the art, including adenoviruses, herpes viruses, and poxviruses such as vaccinia. Although adenovirus type 5 of subgroup C is the most commonly used, adenoviruses include a number of different subgroups. Numerous adenoviruses of human, non-human mammalian, and avian origin are known and are available from depositary institutions such as ATCC. Viruses of the herpes family include, for example, herpes simplex viruses (HSV) and Epstein-Barr viruses (EBV), as well as cytomegaloviruses (CMV) and pseudorabies viruses (PRV). These are also available from depositary institutions such as ATCC.

"Helper virus function(s)" refers to function(s) encoded in the helper virus genome that allow AAV replication and packaging (along with other requirements for replication and packaging described herein). As described herein, the "helper virus function" may be provided in various ways, such as by providing a helper virus or, for example, by providing a polynucleotide sequence encoding essential function(s) to a producer cell in a trans manner. For example, a plasmid or other expression vector containing a nucleotide sequence encoding one or more adenovirus protein is transfected into a producer cell along with an rAAV vector.

In an exemplary embodiment, the AAV1 vector according to the present disclosure has an improved transduction profile for spinal cord astrocytes as compared to an AAV1 vector containing the wild-type capsid protein. That is to say, the AAV1 vector according to the present disclosure has the ability to target spinal cord astrocytes (e.g., astrocyte tropism).

As used herein, the term "tropism" refers to the specificity of the AAV capsid protein present in an AAV virus particle for infecting or transducing a specific type of cell or tissue.

The tropism of the AAV capsid toward a specific type of cell or tissue can be determined by measuring the ability of an AAV vector particle, including the AAV1 capsid protein, to infect or transduce a specific type of cell or tissue using standard analytical methods well known in the art, such as those disclosed in the examples of the present disclosure.

That is to say, the "tropism" means the ability of an AAV vector or virion to infect one or more specific cell type, but may also include the ability of the vector to transfect cells into one or more specific cell type. That is to say, the tropism means the preferential introduction of an AAV vector or virion into a specific cell or tissue type(s) and/or the expression of a sequence carried by the AAV vector or virion in the cell (e.g., transcription and, in some cases, translation), after a preferential interaction with the cell surface that facilitates entry into a specific cell or tissue type(s), in some cases specifically in the cell, for example, the expression of a heterogeneous nucleotide sequence(s) in the case of a recombinant virus.

The term "transduction" as used in the present disclosure refers to the ability of an AAV vector or virion to infect one or more specific cell types. That is to say, the transduction means introducing an AAV vector or virion into a cell and delivering the genetic material contained within the AAV vector or virion into the cell to achieve expression from the vector genome. In some cases, though not all, transduction and tropism may be correlated.

The AAV described herein includes amino acid modifications that confer new or enhanced tissue tropism properties to one or more capsid protein. The AAV1 variant according to the present disclosure targets astrocytes in the spinal cord region.

The term "astrocyte tropism in the spinal cord" used in the present disclosure means tropism toward astrocytes in the spinal cord.

In some exemplary embodiments, the astrocyte tropism in the spinal cord of a peptide-modified hybrid AAV capsid protein is increased by at least 5%, 10%, 20%, 30%, 40%, 50% or more as compared to the astrocyte tropism in the spinal cord of the wild-type AAV capsid protein without the peptide.

In addition, the present disclosure relates to a pharmaceutical composition containing the recombinant AAV1 vector. The composition may further contain a pharmaceutically acceptable carrier.

The "pharmaceutically acceptable carrier" includes any substance that, when combined with the active ingredient of a composition, enables the ingredients to retain their biological activity without causing adverse physiological reactions, such as unintended immune responses. Pharmaceutically acceptable carriers include water, phosphate-buffered saline, emulsions such as oil/water emulsions, and wetting agents. Compositions containing these carriers are formulated by well-known conventional methods, such as those described in Remington's Pharmaceutical Sciences, current ed., Mack Publishing Co., Easton Pa. 18042, USA; A. Gennaro (2000) "Remington: The Science and Practice of Pharmacy", 20th edition, Lippincott, Williams, & Wilkins; Pharmaceutical Dosage Forms and Drug Delivery Systems (1999) H. C. Ansel et al., 7th ed., Lippincott, Williams, & Wilkins; and Handbook of Pharmaceutical Excipients (2000) A. H. Kibbe et al., 3rd ed., Amer. Pharmaceutical Assoc..

In an exemplary embodiment, the spinal cord injury-related conditions may include spinal cord injury caused by trauma, inflammatory spinal cord injury, vascular spinal cord injury, tumor-induced spinal cord injury, etc.

As used in the present disclosure, the term "treatment" refers to any type of intervention or process performed on a subject or the administration of an active agent to a subject for the purpose of reversing, alleviating, improving, inhibiting, delaying, or preventing the progression, development, severity, or relapse of a disease-related syndrome, complication, symptom, or biochemical sign. The treatment may be performed on a subject with the disease or a subject without the disease (e.g., for prophylactic purposes).

**In** addition, in an exemplary embodiment, the present disclosure encompasses a method for preventing or treating a spinal cord injury-related disease such as traumatic spinal cord injury, inflammatory spinal cord injury, vascular spinal cord injury, and tumor-induced spinal cord injury, which includes a step of administering a pharmaceutical composition containing a therapeutically effective amount of the recombinant AAV1 vector to a subject.

The term "administration" as used in the present disclosure refers to the physical introduction of a therapeutic agent or a composition containing a therapeutic agent into a subject using any of various methods and delivery systems known to those skilled in the art. Specific routes of administration for the antibody described in the present disclosure include airway, intravenous, intraperitoneal, intramuscular, subcutaneous, spinal, intravitreal, or other parenteral administration routes, such as by injection or infusion. The phrase "parenteral administration" as used in the present disclosure generally refers to a method of administration other than intestinal and local administration by injection, and is not limited thereto, but includes airway, intravenous, intraperitoneal, intramuscular, intra-arterial, intrathecal, intralymphatic, intralesional, intracapsular, intraorbital, intracardiac, intradermal, transcanal, subcutaneous, subcutaneous, intravitreal, intra-articular, subcapsular, subarachnoid, intramedullary, epidural, and intrasternal injections and infusions, as well as in vivo electroporation. **In** a specific embodiment, it was confirmed that the AAV vector of the present disclosure enables gene delivery to astrocytes in a spinal cord injury animal model by administering it using a micropipette in animal experiments using rats with contusive spinal cord injury.

The term "therapeutically effective amount" as used in the present disclosure refers to the amount of a drug, either alone or in combination with another therapeutic agent, that is effective in "treating" a subject's disease or disorder or reducing the risk, potential, likelihood, or occurrence of the disease or disorder (e.g., spinal cord injury-related disease). The "therapeutically effective amount" includes the amount of a drug or therapeutic agent that provides some improvement or benefit to a subject who has or is at risk of having a disease or disorder (e.g., spinal cord injury disclosed in the present disclosure). Accordingly, the "therapeutically effective amount" is an amount that reduces the risk, potential, likelihood, or occurrence of a disease or disorder, or provides some relief or alleviation, and/or reduces at least one indicator (e.g., spinal cord injury-related disease), and/or reduces at least one clinical symptom of the disease or disorder.

The term "subject" as used in the present disclosure includes any human or non-human animal. The term "non-human animal" includes all vertebrates, e.g., mammals and non-human primates, sheep, dogs, cattle, and non-mammals such as chickens, amphibians, reptiles, etc.

In addition, the present disclosure encompasses a gene delivery vehicle including the recombinant AAV1 vector. The composition may further contain a known pharmaceutically acceptable carrier.

The AAV1 vector of the present disclosure exhibits tropism toward spinal cord astrocytes and has the ability to specifically express genes in the spinal cord astrocytes. Therefore, it can be used as a vector to deliver genes to the spinal cord astrocytes.

### MODE FOR DISCLOSURE

The present disclosure is described in more detail below through examples according to the present disclosure. However, the scope of the present disclosure is not limited by the examples presented below.

### [Examples]

### Example 1: Screening of AAV1 capsid protein variant using AAV library

### 1) Construction of AAV1 variant plasmid library

Random mutations were introduced into the wild-type AAV1 cap gene using error-prone PCR based on wild-type AAV1, which is commonly used in researches targeting central nervous system diseases. During the error-prone PCR process, various concentrations of manganese(II) chloride (Sigma-Aldrich, St. Louis, MO; final concentrations in the total PCR mixture were 0.05 mM, 0.15 mM, and 0.3 mM) and dNTP ratios (2 mM ATP, 2 mM GTP, 1 mM TTP, 1 mM CTP) were used in the cap gene amplification process, resulting in random mutations in each cap gene. Each mutated cap gene was extended using Taq polymerase (Thermo Fisher Scientific, Waltham, MA). The forward primer used was 5'-GCGGAAGCTTCGATCAACTACGC-3' (SEQ ID NO: 9) and the reverse primer used was 5'-GGGCGGCCGCAATTACAGATTACGAGTCAGGTATCTGGTG-3' (SEQ ID NO: 10). Each error-prone PCR product of rep2 downstream, flanked by ITRs (inverted terminal repeats), was cloned to generate a plasmid library containing the cap gene with random mutations added.

### 2) Generation of model of rats with contusive spinal cord injury

The generation of a contusive spinal cord injury model and animal experiments were performed in accordance with the approved protocol (No. 2022-0044) of the Institutional Animal Care and Use Committee of Yonsei University College of Medicine and in compliance with the Animal Care and Use Guidelines issued by the National Institutes of Health.

Adult male Sprague-Dawley rats weighing 240 ± 22 g (7 weeks old) were used to minimize the potential effects of differences in hormone and sex on functional and histological results. The rats were maintained on a 12-hour light-dark cycle and allowed unrestricted food intake. The rats were anesthetized by intraperitoneal injection of 4.24 mg/kg xylazine (Bayern Korea, Seoul, Korea), 97.5 mg/kg ketamine (Yuhan Co., Seoul, Korea), and 1 mg/kg acepromazine (Samu Median, Seoul, Korea).

Under anesthesia, an incision was made along the posterior midline and the lateral thoracic muscles were removed to expose the thoracic vertebrae. Then, under sterile conditions, a vertebroplasty was performed at T9 to expose the spinal cord. The spinal cord was contused using the IH (Infinite Horizon) impactor device, and a force of 200 kdyn was applied to the dorsal surface of the spinal cord. Virus was injected 7 days after the spinal cord injury. The rats were anesthetized again in the same manner as above, the discectomy site was exposed again, and the virus was injected into the left and right sides of the center of the lesion using a glass micropipette (diameter: 0.3 mm). The needle was inserted 1.0 mm deep into the spinal cord from the dorsal surface, and each injection took 1 minute or longer. To prevent cell leakage through the injection track, the needle was kept in place for 1 minute after each injection.

All surgical sites were sutured to the inner layer (muscle layer) and stapled to the outer layer (skin), and 50 mg/kg of cefazolin (Chong Kun Dang Pharmaceutical, Seoul, Korea) was injected intraperitoneally into all rats to prevent bacterial infection. The bladders of the injured rats were emptied manually twice a day until the animals were sacrificed.

### 3) Construction of recombinant AAV1 variant library

A combination of the AAV1 variant plasmid library, a Bluescript plasmid (Stratagene, La Jolla, CA), and an adenovirus helper plasmid (pHelper; Stratagene, La Jolla, CA) was suspended in a 2.5 M CaCl₂ solution and combined slowly with a 2x HEPES buffer solution containing 280 mM NaCl, 1.5 mM Na₂HPO₄ and 50 mM HEPES (pH 7.1). Transfection was performed by adding the final mixture dropwise to HEK293T cells cultured in a 15-cm tissue culture plate. 72 hours after transfection, the HEK293T cells were harvested and lysed through three freeze-thaw cycles, and the lysate was treated with benzonase II (Sigma-Aldrich; 1 unit/mL) to remove cellular genomic contaminants. The viral vector was purified by iodixanol density gradient ultracentrifugation (OptiPrep, Alere Technologies AS, Oslo, Norway) using a Vti65.2 rotor (Beckman Coulter; 42,000 rpm). The purified AAV vector was buffer-exchanged with a 1x PBS solution containing 0.01% (v/v) Tween-20 (Sigma-Aldrich) using an Amicon tube (Merck Millipore, Billerica, MA; molecular weight cutoff: 100 kDa). Finally, the genomic titer of the DNA-resistant AAV vector was determined by quantitative PCR (Mini Opticon, Bio-Rad, Hercules, CA).

### 4) Screening of AAV1 capsid protein variants

The recombinant AAV1 variant library was injected into a 200 kdyn contusion spinal cord injury rat model prepared according to the method described above on day 5. One week after virus transplantation, spinal cord injury sites were isolated, and only astrocytes were selected from the lesions using the MACS sorting method (MACS sorting is a widely used method for isolating specific cells, which is a method of separating cells using a magnet and antibodies that bind to specific cell surface markers.).

Viral genes were recovered from isolated astrocytes using the QIAamp DNA mini kit (Qiagen 51306), and the DNA was amplified by PCR. Capsid genes from a selected library were then read using the Pacbio-SMRT (Pacific Bioscience) next-generation sequencing (NGS) instrument. As a result of the NGS, mutation information of variants originating from AAV1 was collected. Among them, the top 8 variants with more than 100 NGS copies were found to contain mutations D595H, D712E, and F713Y, as well as mutations K315R, F584L, A598V, and N642H, in the amino acid sequence of the wild-type AAV1 capsid protein, represented by SEQ ID NO: 1 (Table 1). Table 1 below shows the variants found as a result of the NGS of the spinal cord injury model.

**[Table 1]**

| No. | serotype | Point mutation | Copy |
|---|---|---|---|
| 1 | >AAV1 | D595H, D712E, F713Y | 11070 |
| 2 | >AAV1 | K315R, F584L, A598V, N642H | 5319 |
| 3 | >AAV1 | K315R, D595H, D712E, F713Y | 792 |
| 4 | >AAV1 | F584L, A598V, N642H | 641 |
| 5 | >AAV1 | D595H | 227 |
| 6 | >AAV1 | D712E, F713Y | 164 |
| 7 | >AAV1 | K315R | 125 |
| 8 | >AAV1 | D712E | 115 |

The copy numbers for each mutation were added for all the variants obtained from the NGS result. When mutations with a high frequency of occurrence in the astrocytes with more than 100 copy numbers were collected, D595H, D712E, F713Y, K315R, F584L, A598V, T194A, A196S, A197G, T201N, S205A, L129F, A224S, N223S, H272T, S268T, T265S, A263G, S262N, and S264T were identified in order in the amino acid sequence of the wild-type AAV1 capsid protein represented by SEQ ID NO: 1. Therefore, it was confirmed that these mutations increase the specificity for astrocytes at the spinal cord injury site. Table 2 below shows the number of the observation of point mutations in the NGS result.

**[Table 2]**

| mutation | copy |
|---|---|
| D712E | 13638 |
| D595H | 13562 |
| F713Y | 13486 |
| K315R | 7172 |
| A598V | 6855 |
| F584L | 6828 |
| N642H | 6828 |
| T194A | 248 |
| A1965 | 243 |
| A197G | 235 |
| T201N | 216 |
| S205A | 209 |
| L129F | 187 |
| A224S | 176 |
| N2235 | 174 |
| H272T | 131 |
| S268T | 129 |
| T265S | 116 |
| A263G | 112 |
| S262N | III |
| S264T | III |

In addition, the cap gene portion was extracted from the PCR product of the selected variant library produced before NGS by treating it with HindIII and NotI restriction enzymes, which was then connected to the pSub2 backbone plasmid and transfected into *E. coli.* After culturing, the resulting single colony was randomly selected and then subjected to Sanger sequencing. As a result, in the amino acid sequence of the wild-type AAV1 capsid protein represented by SEQ ID NO: 1, two variants containing mutations of D595H, D712E, F713Y and K315R, F584L, A598V, and N642H were found with the highest frequency, appearing 4 and 2 times, respectively, which matched the rankings in the NGS result, and these were named AAVSN2 and AAVSN3, respectively. Gene clones of each AAV variant selected from the astrocytes at the top spinal cord injury site were constructed based on the NGS and Sanger sequencing results. After selecting astrocyte tropism mutations at the spinal cord injury site from the library pool, each cap gene obtained using HindIII/NotI restriction enzymes was used to construct recombinant AAV1 variants loaded with CMV-GFP or NeuroD1-P2A-GFP genes using AAVSN2 and AAVSN3 gene clones into which HindIII and NotI were introduced.

Subsequently, the recombinant AAV1 variant (AAVSN2 variant) was injected into the animal model of contusive spinal cord injury, and it was confirmed that it could induce high gene expression efficiency in the astrocytes at the contusive spinal cord injury site.

In addition, it was confirmed that the recombinant AAV1 variant (AAVSN3 variant) has a high specific infection efficiency in the astrocytes at the contusive spinal cord injury site.

### 5) Construction of recombinant AAV1 vectors (AAV1 SN2 and SN3 variants)

### ① Construction of packaging plasmid variants

The cap genes of each of the AAVSN2 and AAVSN3 variants obtained from Sanger sequencing were subcloned into pXX2 (UC Berkeley, David Schaffer Lab) in which HindIII and NotI were introduced, and the construction of astrocyte-specific plasmid variants for carrying the CMV-GFP gene and genes such as CMV-GFP, NeuroD1-P2A-GFP, etc. was completed.

### ② Introduction of plasmids into astrocytes

### Construction of AAV1 SN2 and SN3 variants

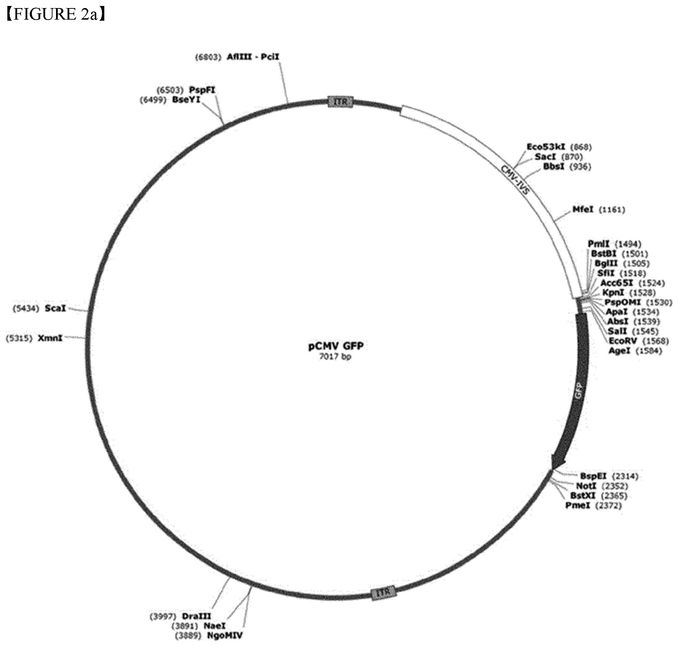

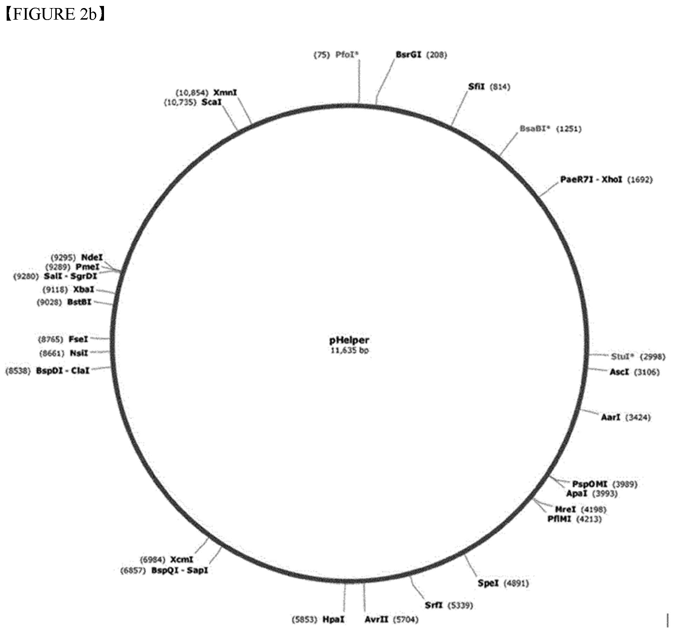

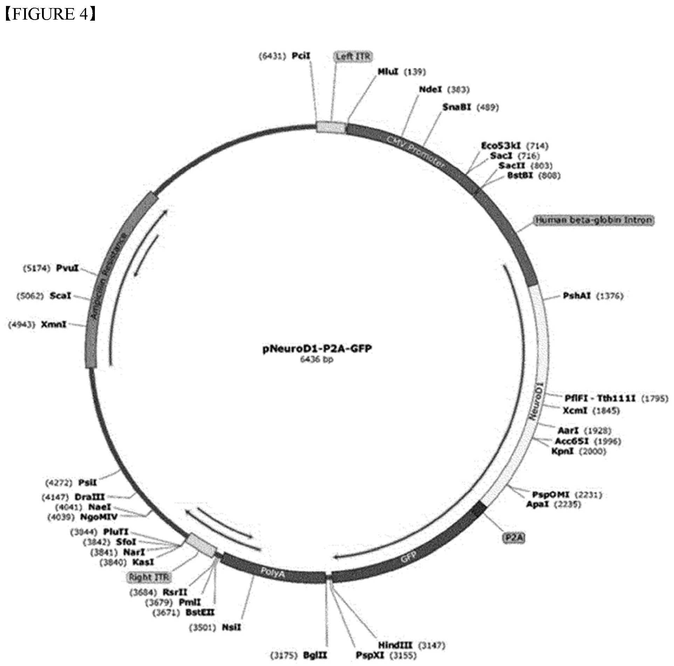

17 of the constructed AAVSN2 or AAVSN3 variant plasmid, 17 of a cell-specific reporter gene (pCMV-GFP [FIG. 2A] or pNeuroD1-P2A-GFP [FIG. 4]), and 17 of roD1-P2A-G [FIG. 2B] were transfected into HEK293T cells as a calcium-phosphate complex. After 72 hours, the HEK293T cells were harvested and lysed through three freezing-thawing cycles. Afterwards, the cell debris was removed by centrifugation, and nucleic acids from the virus-producing cells were removed by incubating with 10 U/mL benzonase at 37 °C for 30 minutes.

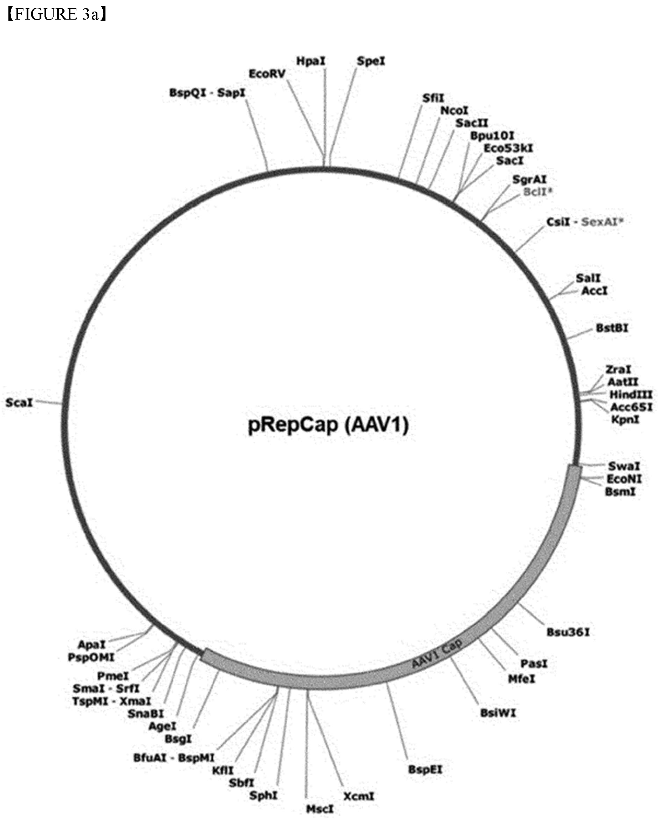

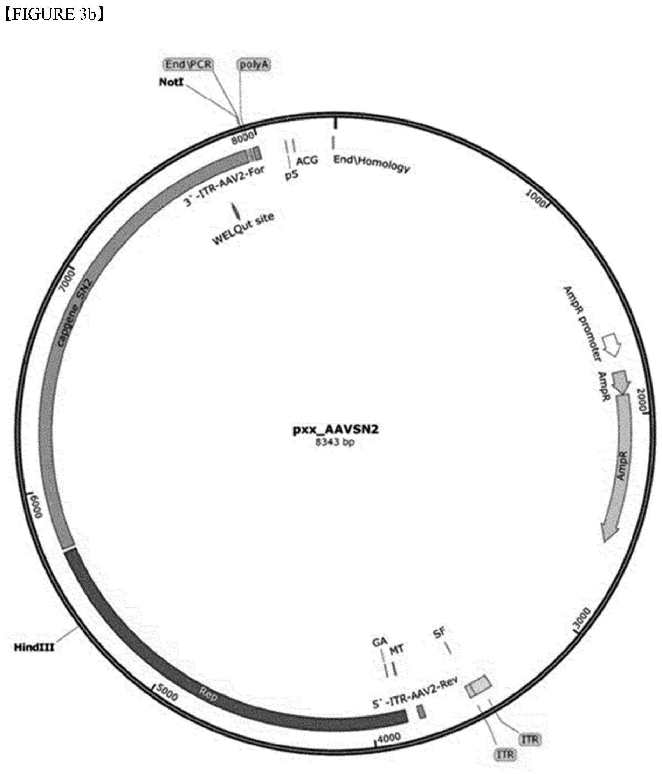

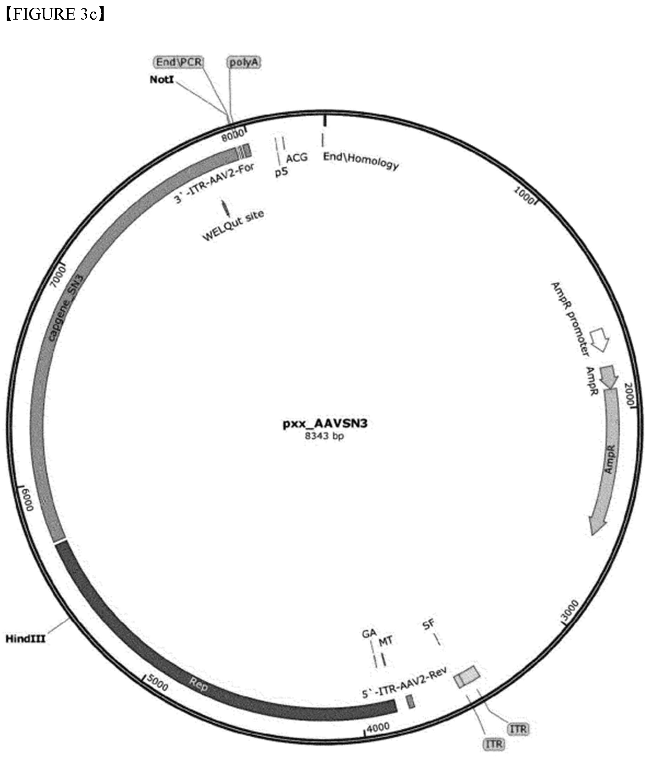

The cleavage maps of the constructed AAVSN2 and AAVSN3 variant plasmids are shown in FIG. 3A, FIG. 3B and FIG. 3C. The complete nucleotide sequences of the AAV1 SN2 and SN3 variant plasmids are SEQ ID NO: 7 and SEQ ID NO: 8, respectively.

### ③ Construction of wild-type AAV1

17 µg of a packaging plasmid containing the rep gene of AAV2 and the cap gene of AAV1, 17 µg of an ITR flanked reporter gene (pCMV-GFP, pCMV-LacZ or pCMV-FGF12-IRES-GFP), and 17 µg of pHelper were transfected into HEK293T cells as a calcium-phosphate complex, and after about 48 hours, only the cell pellet was collected and the AAV inside the cells was extracted by freezing and thawing. Afterwards, the cell debris was removed by centrifugation and nucleic acids from the virus-producing cells were removed by incubating with 10 U/mL of benzonase at 37 °C for 30 minutes.

### 6) Purification of wild-type AAV1 and AAVSN2 and AAVSN3 variants

The AAV solution was purified by iodixanol density gradient ultracentrifugation (OptiPrep, Alere Technologies AS, Oslo, Norway) using a Vti65.2 rotor (Beckman Coulter; 42,000 rpm). The purified AAV vector was buffer-exchanged with a 1x PBS solution containing 0.01% (v/v) Tween-20 (Sigma-Aldrich) using an Amicon tube (Merck Millipore, Billerica, MA; molecular weight cutoff: 100 kDa). Finally, the genomic titer of the DNase-resistant AAV vector was determined by quantitative PCR (Mini Opticon, Bio-Rad, Hercules, CA).

### 7) Measurement of titers of wild-type AAV1 and AAVSN2 and AAVSN3 variants

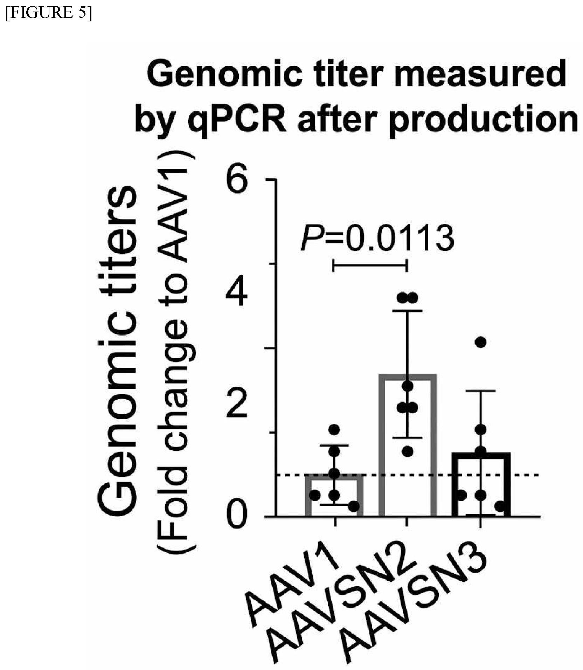

After 12 hours of transduction with AAVSN2-CMV-GFP, AAVSN3-CMV-GFP or AAV1-CMV-GFP, the medium was changed to 1% PS DMEM supplemented with 1% FBS, and virus harvesting was performed at 60 hours after the transduction. Viral titers were determined by qPCR (FIG. 5).

AAVSN2-GFP showed a higher titer than the wild-type AAV1-GFP, which has good packaging efficiency, with statistically significant difference. This means that the AAVSN2 variant is easier to produce than the wild-type AAV1.

AAVSN3-GFP was found to have no statistically significant difference from the wild-type AAV1-GFP, which has good packaging efficiency. This means that the AAVSN3 variant is as easy to produce as the wild-type AAV1.

### Example 2: Confirmation of infection by recombinant AAV1 variant virus

### 1) In vitro experiment

Since astrocytes could not be isolated from the primary culture obtained from the spinal cord of 7-week-old male Sprague-Dawley rats, primary mixed glial cells including astrocytes were seeded into a 24-well plate at 2x10⁵ cells. 12 hours later, AAVSN3-CMV-GFP and AAV1-CMV-GFP infections were performed at MOIs of 10,000 and 50,000. Three days after the infection, trypsin was treated, and single cells were tagged with Alexa 488 and Alexa 647 fluorescent dyes using a GFP antibody (Alexa Fluor^{®} 488-conjugated anti-GFP antibody (1:500 dilution; Thermo Fisher Scientific)) and a GFAP antibody (Alexa Fluor^{®} 647-conjugated anti-GFAP antibody (1:500 dilution; BioLegend)), respectively, followed by flow cytometry analysis.

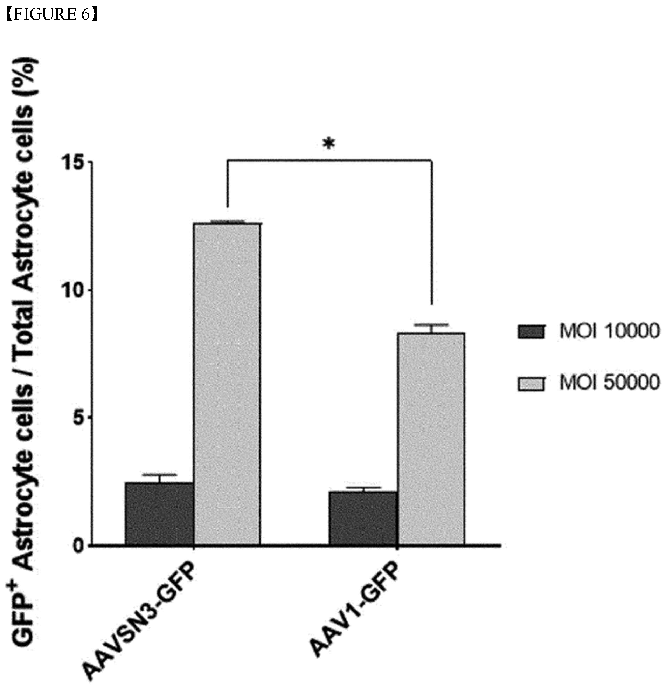

Through this, astrocytes with strong GFAP signaling were isolated, and the proportion of the cells in which the GFP gene was expressed by AAV was measured. At MOI of 10000, there was no statistically significant difference, but at MOI of 50000, it was confirmed that AAVSN3-GFP exhibited a statistically significant higher viral infection efficiency in primary astrocytes than AAV1-GFP (FIG. 6).

### 2) In vivo experiment - verification of viral genome

7-week-old male Sprague-Dawley rats were injected with AAVSN2, AAVSN3, or wild-type AAV1 (5x10⁸ vg/µL; 6 µL) using a micropipette at a force of 200 kdyn 5 days after contusive spinal cord injury (acute phase) (At this time, the injected virus carried the GFP gene, and the expression of the gene could be confirmed through GFP expression.).

Two weeks after the AAV transplantation, perfusion was performed to remove blood, and the spinal cord including the injury site was dissected and recovered. DNA was recovered from the tissue and the viral genome was measured by qPCR.

Each viral genome value was analyzed after being normalized to GAPDH.

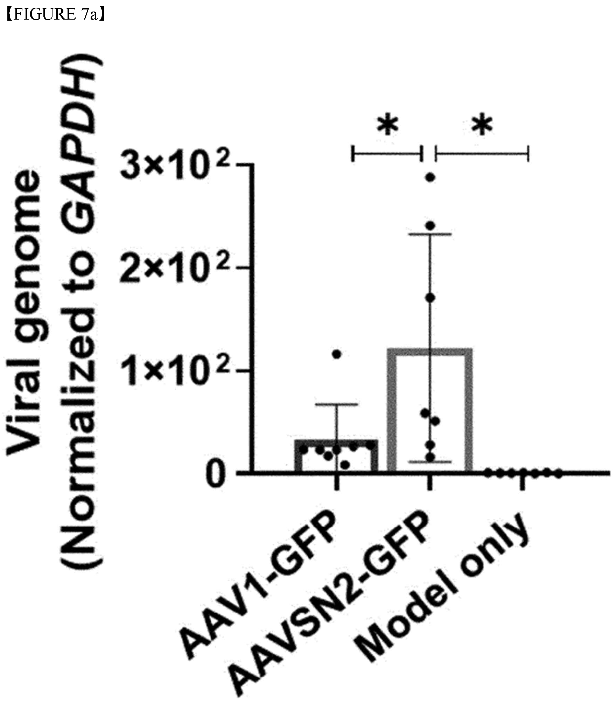

When compared to AAV1-GFP, AAVSN2-GFP was found to have a statistically significant increase in the viral genome within the spinal cord tissue including the injury site. In other words, it was confirmed that AAVSN2 infects the spinal cord injury site more than the wild-type AAV1 (FIG. 7A).

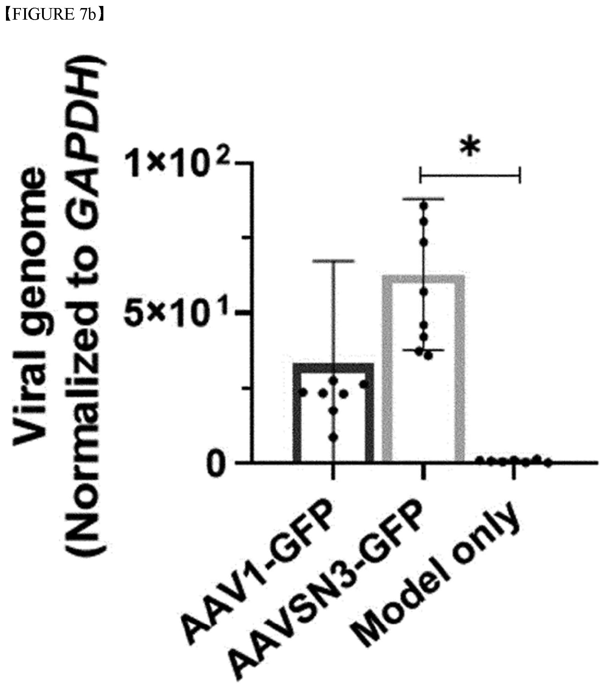

In addition, while the viral genome of AAV1-GFP in the spinal cord tissue including the injury site did not show a statistically significant difference compared to the model only, it was confirmed that the viral genome of AAVSN3-GFP increased significantly as compared to the model only. In other words, it was confirmed that AAVSN3 infects the spinal cord injury site more than the wild-type AAV1 (FIG. 7B).

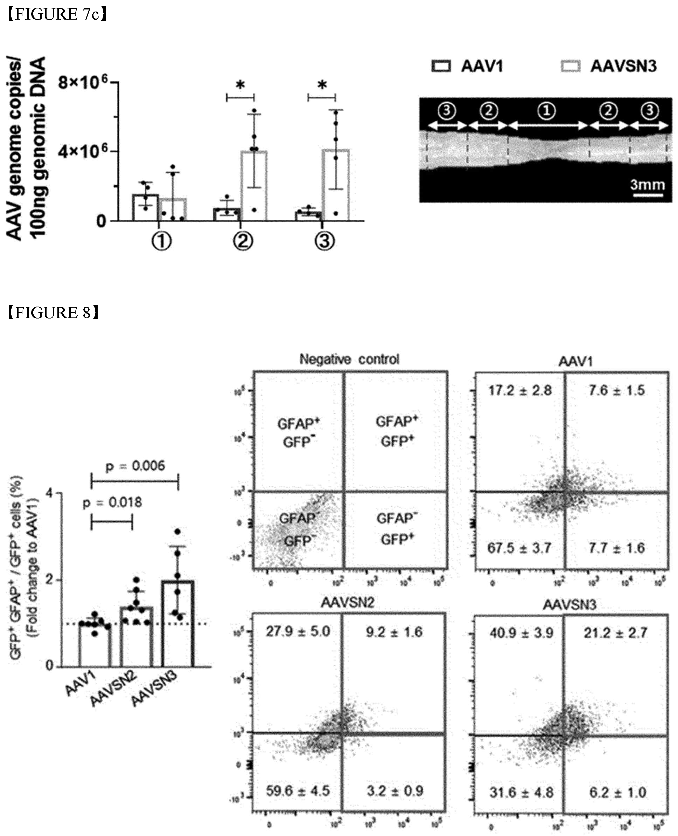

Meanwhile, the number of AAV genome copies per 100 ng of genomic DNA recovered from each tissue was analyzed. In the central region centered around the injury site, there was no statistically significant difference between the wild-type AAV1 and AAVSN3. In the spinal cord tissues in the regions ② and ③, which are based on the distance from the center, AAVSN3 showed a statistically significant increase as compared to the wild-type AAV1. In other words, it was shown that AAVSN3 can infect the whole spinal cord area, including the injury site, as compared to the wild-type AAV1 (FIG. 7C).

### 3) In vivo experiment - confirmation of gene delivery ability to astrocytes

7-week-old male Sprague-Dawley rats were injected with AAVSN2, AAVSN3, or wild-type AAV1 (5x10⁸ vg/µL; 6 µL) using a micropipette into the center of the spinal cord injury at a force of 200 kdyn 5 days after contusive spinal cord injury (acute phase) (At this time, the injected virus carried the GFP gene, and the expression of the gene could be confirmed through GFP expression.).

Two weeks after the AAV transplantation, perfusion was performed to remove blood, and the spinal cord including the injury site was dissected and recovered. Single cells were isolated using a neural tissue isolation kit.

For AAVSN2, single cells were tagged with Alexa 488 and rhodamine fluorescent dyes using GFP and GFAP antibodies, respectively, and then flow cytometry was performed. A sample with the AAV vector not administered to the lesion site of rats with contusive spinal cord injury was designated as model only. A sample that was not tagged with GFP or GFAP antibodies for model only was designated as GFP(-)GFAP(-), and a sample that was tagged with GFP or GFAP antibodies for model only was designated as GFP(-)GFAP(+) for gating for GFAP. Gating for GFP was performed on GFP(-) and GFP(+).

The proportion of astrocytes among the GFP-expressing cells shown in FIG. 8 was calculated from (GFP+ astrocytes) / ((GFP+ astrocytes) + (cells other than GFP+ astrocytes)). When the proportion of astrocytes among all GFP-expressing cells was plotted, AAVSN2-GFP and AAVSN3-GFP showed a higher proportion than AAV1-GFP, confirming that AAVSN2-GFP and AAVSN3-GFP have higher gene delivery efficiency specifically to astrocytes as compared to other cells within the spinal cord lesion site, as compared to AAV1-GFP [FIG. 8].

### 4) In vivo experiment - confirmation of gene expression intensity in astrocytes

7-week-old male Sprague-Dawley rats were injected with AAVSN2, AAVSN3, or wild-type AAV1 (5x10⁸ vg/µL; 6 µL) using a micropipette into the center of the spinal cord injury at a force of 200 kdyn 5 days after contusive spinal cord injury (acute phase) (At this time, the injected virus carried the GFP gene, and the expression of the GFP gene could be confirmed through immunohistochemical staining. Mock viruses carried the GFP gene.)

At 8 weeks after the AAV transplantation, perfusion was performed to remove blood, the tissue was fixed with 4% PFA, and sections were prepared using a cryostat. The distribution of AAV and gene expression intensity in the central nervous system were analyzed using immunochemical staining with GFP on the sectioned samples.

It was confirmed that AAVSN2-GFP, as compared to AAV1-GFP, showed specific gene expression around the injury site.

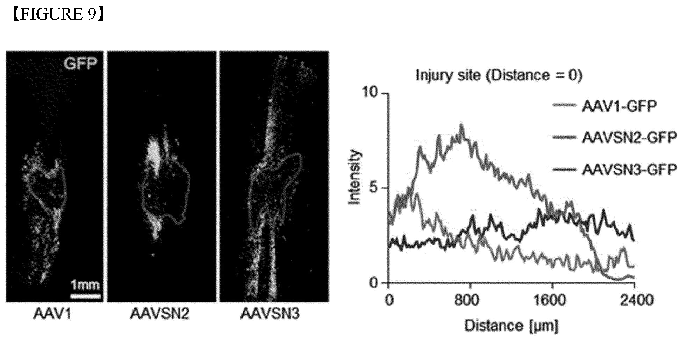

With the injury site set to distance = 0, the expression intensity was confirmed as shown in the graphs of FIG. 9. It was confirmed that the use of AAVSN2 was more effective than the wild-type AAV1 for intensive treatment of the spinal cord injury site.

In addition, as compared to AAV1-GFP, AAVSN3-GFP was found to show overall gene expression over a wider area relative to the injury site.

With the injury site set to distance = 0, the expression intensity was confirmed as shown in the graphs of FIG. 9. It was confirmed that the use of AAVSN3 was more effective than the wild-type AAV1 for overall spinal cord treatment.

### Example 3: Confirmation of gene delivery ability recombinant AAV1 variant in astrocytes around spinal cord

7-week-old male Sprague-Dawley rats were injected with AAVSN2, AAVSN3, or wild-type AAV1 (5x10⁸ vg/µL; 6 µL) using a micropipette into the center of the spinal cord injury at a force of 200 kdyn 5 days after contusive spinal cord injury (acute phase) (At this time, the injected virus carried the GFP gene, and the expression of the gene could be confirmed through GFP expression. Mock viruses carried the GFP gene.)

At 8 weeks after the AAV transplantation, perfusion was performed to remove blood, and tissues were fixed with 4% PFA and sections were taken using a cryostat. The distribution of immune cells in the central nervous system was analyzed by immunochemical staining using GFP and GFAP on the sectioned samples.

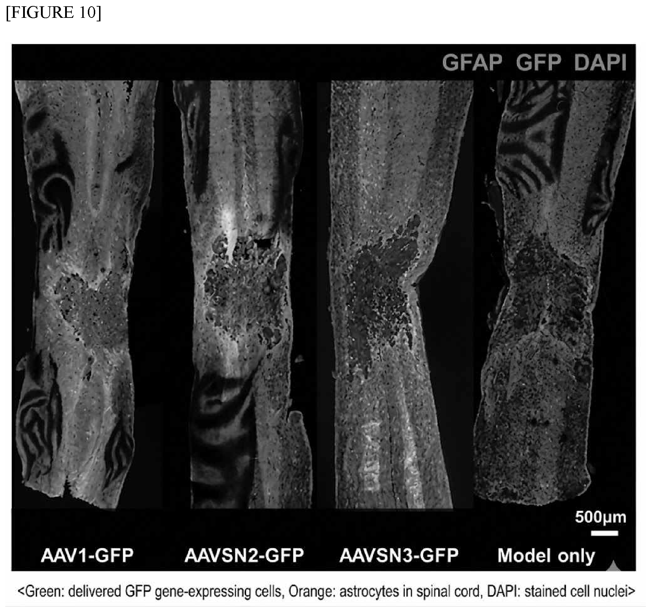

As a result of examining the spinal cord around the spinal cord injury site, it was confirmed that the AAV1-based AAVSN2 or AAVSN3 showed high gene delivery efficiency to the spinal cord injury site as compared to the wild-type AAV1, with the induction of a strong GFP signal concentrated at the spinal cord injury site [FIG. 10].

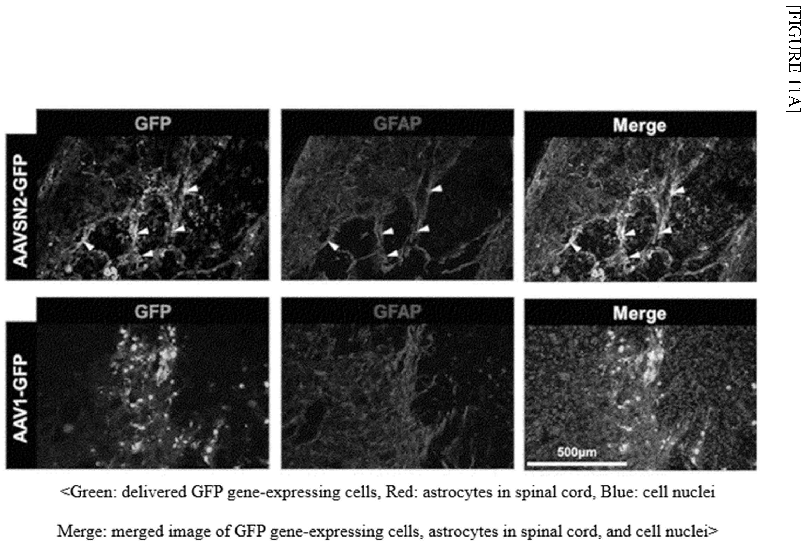

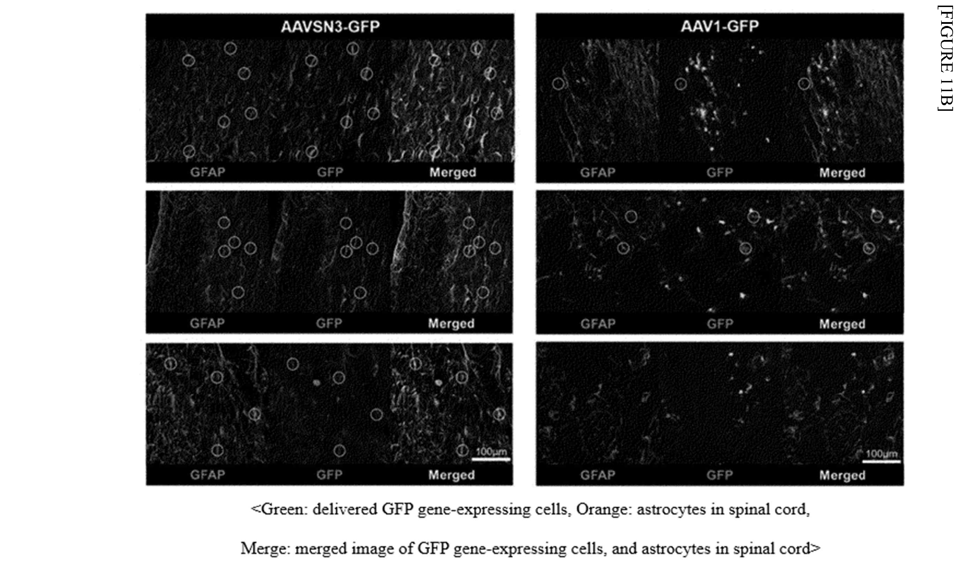

In addition, high-magnification examination of the area near the lesion site revealed that both AAVSN2 and AAVSN3 exhibited a GFP signal pattern similar to that of the GFAP signal and had more sites of co-expression of both signals as compared to the wild-type AAV1. This confirmed that AAVSN2 or AAVSN3 exhibited a higher infection rate of astrocytes as compared to the wild-type AAV1 [FIG. 11].

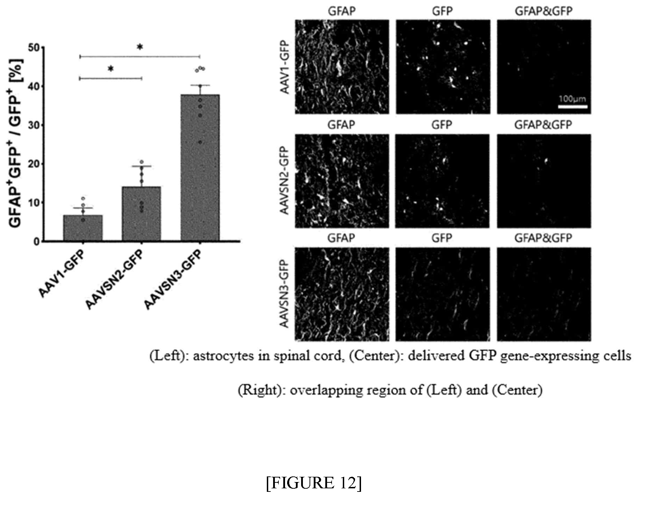

Furthermore, high-magnification images were analyzed using the ImageJ program to measure the ratio of GFAP+ cells among GFP+ cells. It was confirmed that AAVSN2 and AAVSN3 had a higher delivery rate to astrocytes at the contusive spinal cord injury site than the wild-type AAV1 [FIG. 12].

### Example 4: Confirmation of gene delivery ability of recombinant AAV1 variant to astrocytes and other cells near spinal cord

7-week-old male Sprague-Dawley rats were injected with AAVSN2, AAVSN3, or wild-type AAV1 (5x10⁸ vg/µL; 6 µL) using a micropipette at a force of 200 kdyn 5 days after contusive spinal cord injury (acute phase) (At this time, the injected virus carried the GFP gene, and the expression of the gene could be confirmed through GFP expression. The mock virus carried the GFP gene.)

At 8 weeks after the AAV transplantation, perfusion was performed to remove blood, and after fixing the tissue with 4% PFA, sections were performed using a cryostat. The distribution of immune cells within the central nervous system was analyzed by immunochemical staining using GFP, GFAP, Tuj1 (rabbit monoclonal ant-Tuj1 antibody (1:2000 dilution; Covance)), and Iba1 (rabbit anti-Iba1 antibody (1:500 dilution; Wako Pure Chemical Industries), respectively, for the sectioned samples (GFAP: astrocytes, Tuj1: neurons, Iba1: microglia).

IHC images were analyzed using the ImageJ program to quantify the percentage of signals (GFAP, Tuj1, Iba1) from GFP+ cells.

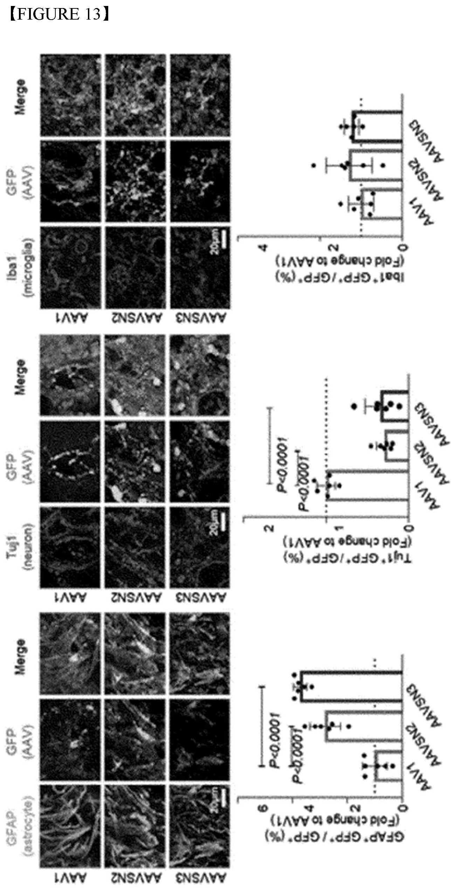

When compared to AAV1-GFP, AAVSN2-GFP showed increased GFP expression in astrocytes and decreased GFP expression in neurons. There was no statistically significant difference in microglia. This indicates that AAVSN2 reverses neuronal tropism of the wild-type AAV1 and that tropism toward astrocytes has increased [FIG. 13].

### Comparative Example 2: Confirmation of gene delivery ability of recombinant AAV1 variant to microglia near spinal cord

7-week-old male Sprague-Dawley rats were injected with AAVSN2, AAVSN3, or wild-type AAV1 (1x10⁹ vg/µL; 10 µL) using a micropipette into the center of the spinal cord injury 5 days after contusive spinal cord injury at a force of 200 kdyn (acute phase) (At this time, the injected virus carried the NeuroD1-P2A-GFP gene, and the expression of the GFP gene could be confirmed through immunohistochemical staining. The mock virus carried the GFP gene.)

At 8 weeks after the AAV transplantation, perfusion was performed to remove blood, the tissue was fixed with 4% PFA, and sections were taken using a cryostat. The distribution of immune cells in the central nervous system was analyzed by immunochemical staining using Iba1 (rabbit anti-Iba1 antibody (1:500 dilution; Wako Pure Chemical Industries)).

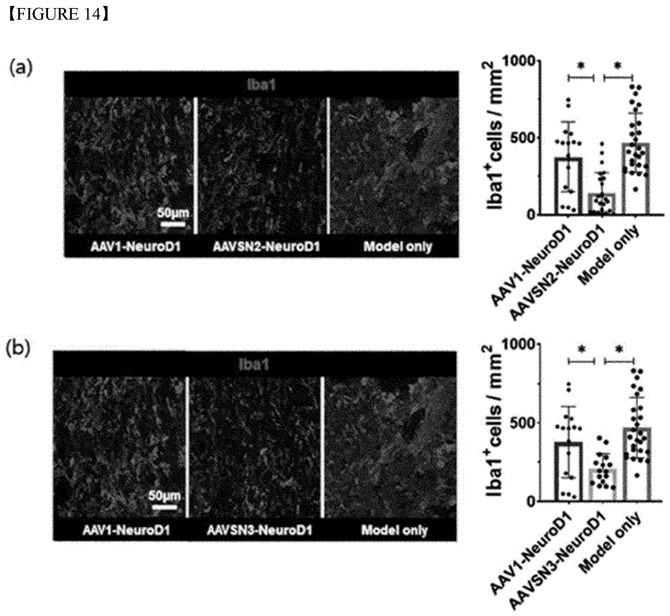

It was confirmed that immune cells were statistically significantly reduced at the spinal cord injury site as compared to AAVSN2-NeuroD1 or AAVSN3-NeuroD1 and AAV1-NeuroD1 or model only. In other words, it was confirmed that gene delivery ability was poor in microglia near the spinal cord [FIG. 14].

### Comparative Example 3: Confirmation of gene delivery ability of recombinant AAV1 variant to tissues other than spinal cord

7-week-old male Sprague-Dawley rats were injected with AAVSN2, AAVSN3, or wild-type AAV1 (5 x 10⁸ vg/µL; 6 µL) using a micropipette at a force of 200 kdyn 5 days after contusive spinal cord injury (acute phase) (At this time, the injected virus carried the GFP gene, and the expression of the gene could be confirmed through GFP expression. Mock viruses carried the GFP gene.).

Two weeks after the AAV transplantation, perfusion was performed to remove blood, and liver, spleen, lung, kidney, brain, and heart tissues were recovered.

DNA was recovered from the tissues, and viral genome values were measured by qPCR.

Each viral genome value was analyzed after normalization to GAPDH.

Although there were no statistically significant differences between the AAV1-GFP and AAVSN2-GFP groups in the liver, spleen, lungs, kidneys, brain, and heart, AAV1-GFP showed a statistically significant increase in the liver and lungs as compared to the model-only group, whereas AAVSN2-GFP showed no statistically significant difference in any tissue as compared to the model-only group. In other words, it was confirmed that the off-target issue to other tissues after transplantation was reduced in AAVSN2-GFP as compared to AAV1-GFP.

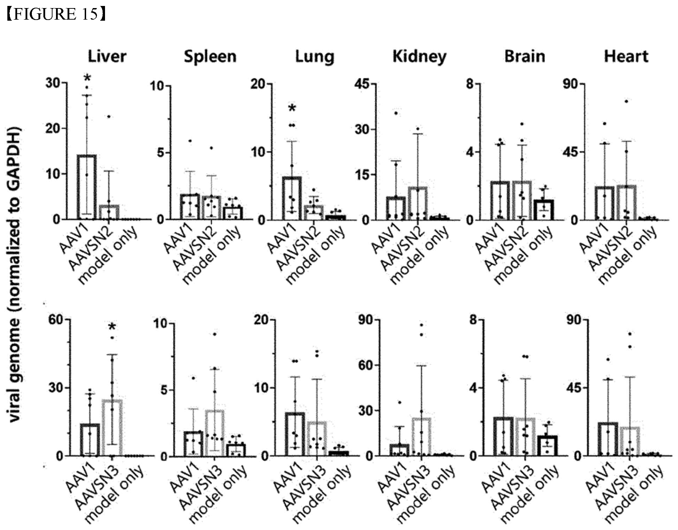

AAVSN3-GFP showed no statistically significant difference in the liver as compared to AAV1-GFP, but showed a statistically significant increase as compared to the model-only group. In the spleen, lungs, kidneys, brain, and heart, there were no statistically significant differences as compared to AAV1-GFP or as compared to the model-only group. In other words, there was no difference in off-target effects as compared to AAV1-GFP, and it was confirmed that there were no statistically significant differences in the spleen, lungs, kidneys, brain, and heart as compared to the model only [FIG. 15].

### Example 4: Confirmation of recovery of spinal cord injury site by recombinant AAV1 variant

7-week-old male Sprague-Dawley rats were injected with AAVSN2 or wild-type AAV1 (1x10⁹ vg/µL; 10 µL) using a micropipette at a force of 200 kdyn 5 days after contusive spinal cord injury (acute phase) (At this time, the injected virus was loaded with the NeuroD1-P2A-GFP gene, which can reprogram astrocytes into neurons, thereby inducing functional improvement of damaged neuronal function. By inserting P2A with the amino acid sequence ATNFSLLKQAGDVEENPGP (SEQ ID NO: 11), GFP was prevented from having a functional effect on the NeuroD1 protein. Immunohistochemical staining was used to confirm gene expression as a GFP signal.).

After the AAV transplantation, a test was conducted at weekly intervals to quantify motor ability based on the movement of three joints (ankle, knee, and hip) on a scale from 0 to 21 (normal). The test was conducted for 8 weeks after confirming that there were no statistically significant differences among the four groups prior to injection. Starting from week 4, statistically significant differences were observed between the AAVSN2-NeuroD1 group (square: left graph) and the model only group (diamond: left graph). There was no statistically significant difference among the remaining groups.

Through a comparison of AAVSN2-NeuroD1 and AAVSN2-GFP, it was confirmed that the NeuroD1 gene actually induces the reprogramming of astrocytes into neurons, thereby inducing functional improvement.

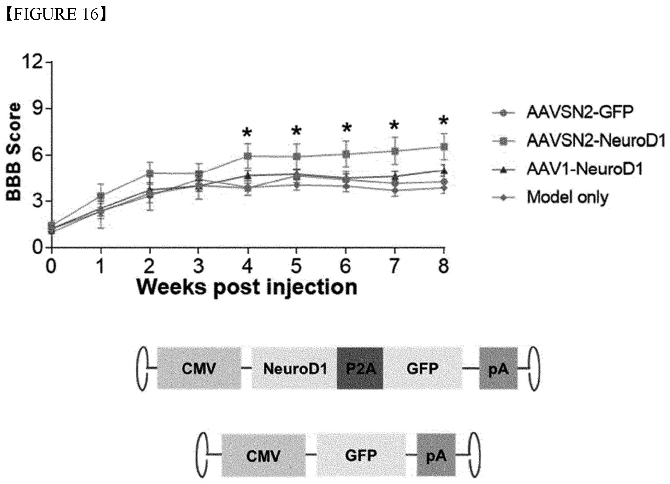

And, through a comparison of AAVSN2-NeuroD1 and AAV1-NeuroD1, it was confirmed that AAVSN2 has a higher efficiency of NeuroD1 gene delivery to astrocytes near the injury site as compared to AAV1, and that only in the case of AAVSN2-NeuroD1, the BBB score increases statistically significantly as compared to model only [FIG. 16].

### Example 5: Confirmation of reprogramming after introduction of therapeutic gene by recombinant AAV1 variant

7-week-old male Sprague-Dawley rats were injected with AAVSN2 or wild-type AAV1 (1x10⁹ vg/µL; 10 µL) using a micropipette at a force of 200 kdyn 5 days after contusive spinal cord injury (acute phase) (At this time, the injected virus was loaded with the NeuroD1-P2A-GFP gene, and the expression of the GFP gene could be confirmed through immunohistochemical staining.).

Perfusion was performed at 3 and 8 weeks after the AAV transplantation to remove blood, and tissues were fixed with 4% PFA and sections were taken using a cryostat. The distribution of AAV in the central nervous system was analyzed by immunochemical staining using GFP and GFAP or Tuj1 (rabbit monoclonal ant-Tuj1 antibody (1:2000 dilution; Covance)).

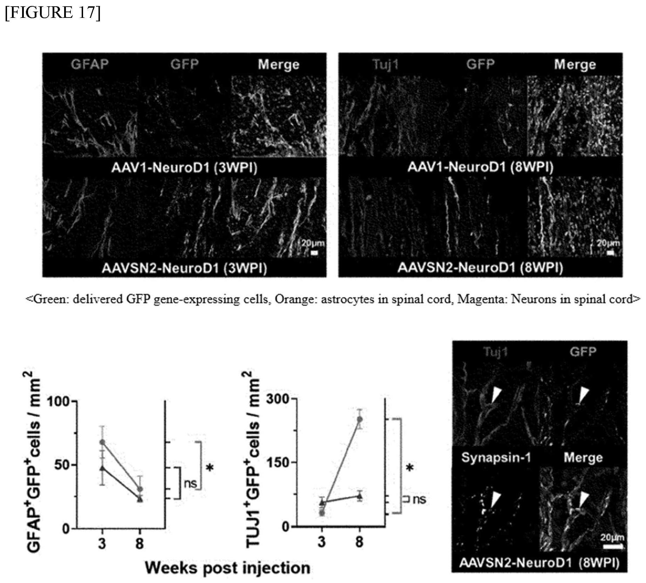

The images of the spinal cord injury site transplanted with AAVSN2-NeuroD1 and AAV1-NeuroD1 are shown at the top of FIG. 17.

It was confirmed that AAVSN2-NeuroD1 exhibited the highest co-expression of GFAP and GFP signals at week 3. At week 8, AAVSN2-NeuroD1 exhibited the highest co-expression of Tuj1 and GFP signals. Quantitative analysis results confirmed that AAVSN2-NeuroD1 was effective in in vivo reprogramming of astrocytes into neurons at the spinal cord injury site, as the co-expression of GFAP+GFP+ decreased statistically significantly at weeks 3 and 8, and the co-expression of Tuj1+GFP+ increased statistically significantly at weeks 3 and 8. When AAVSN2-NeuroD1 was transplanted, it was confirmed via synapsin-1 that the reprogrammed neurons formed synaptic connections [bottom of FIG. 17].

More specifically, the graph on the bottom left of FIG. 17 shows that the number of astrocytes in which GFAP and GFP were co-expressed, i.e., in which the ND1 gene was delivered to the astrocytes via AAV, decreased over time from week 3 to week 8. In contrast, the number of neurons in which Tuj1 and GFP were co-expressed, i.e., in which ND1 was delivered by AAV, was very small at week 3 but increased significantly at week 8. These two phenomena indicate that the astrocytes that received the ND1 gene were reprogrammed into neurons.

In addition, the reason why the signals of synapsin-1 and Tuj1 GFP overlap in the image on the bottom right is that it is an image at week 8 (after treatment) rather than at week 3. After treatment, the co-expression of Tuj 1+GFP+ increases and synapses are formed, so it is desirable data showing the co-expression of Tuj 1+GFP+ signals and synapsin-1.

Thus, it was confirmed that the ND1 gene is delivered to astrocytes and reprogrammed into neurons.

### Example 6: Confirmation of body weight change after introduction of therapeutic gene by recombinant AAV1 variant

7-week-old male Sprague-Dawley rats were injected with AAVSN2, AAVSN3, or wild-type AAV1 (1 x 10⁹ vg/µL; 10 µL) using a micropipette into the center of the spinal cord injury at a force of 200 kdyn 5 days after contusive spinal cord injury (acute phase) (At this time, the injected virus carried the NeuroD1-P2A-GFP gene, and the expression of the GFP gene could be confirmed through immunohistochemical staining. The mock virus carried the GFP gene.)

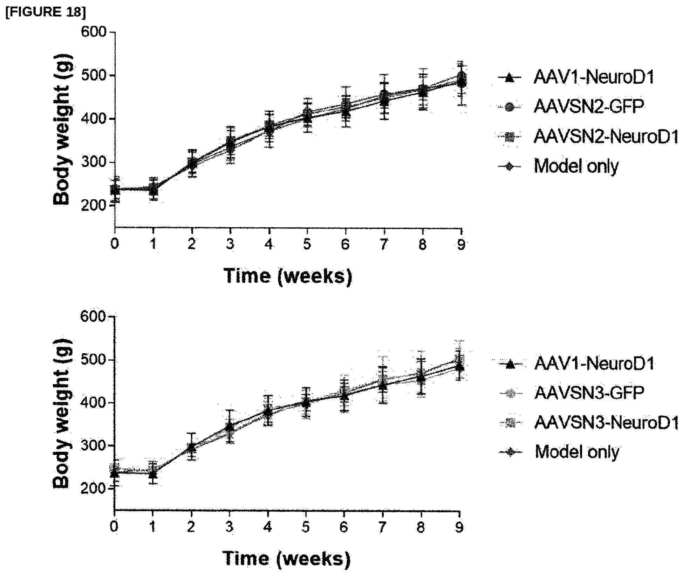

Body weight was measured at 1-week intervals starting from the generation of the spinal cord injury animal model to the AAV transplantation, and it was confirmed that there was no statistically significant difference between all the groups [FIG. 18].

### Example 7: Hematological toxicity test after introduction of therapeutic gene by recombinant AAV1 variant

7-week-old male Sprague-Dawley rats were injected with AAVSN3, AAVSN2, or wild-type AAV1 (1 x 10⁹ vg/µL; 10 µL) using a micropipette into the center of the spinal cord injury at a force of 200 kdyn 5 days after contusive spinal cord injury (acute phase) (At this time, the injected virus carried the NeuroD1-P2A-GFP gene, and the expression of the GFP gene could be confirmed through immunohistochemical staining. The mock virus carried the GFP gene.)

Blood was collected at 8 weeks after the AAV transplantation, plasma was separated, and hematological toxicity test was performed for GPT (glutamic-pyruvic transaminase), GOT (glutamic-oxaloacetic transaminase), and BUN (blood urea nitrogen).

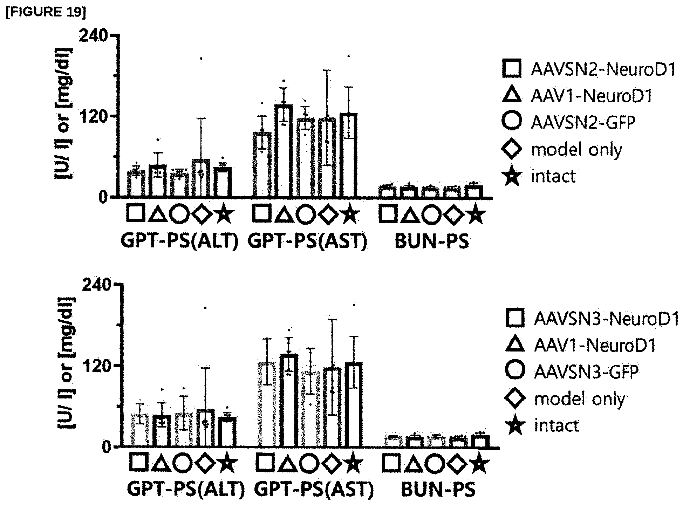

It was confirmed that there was no statistically significant difference between all the groups: AAV1-NeuroD1, AAVSN2-NeuroD1 or AAVSN3-NeuroD1, AAVSN3-GFP, model only, intact [FIG. 19].

## Claims

1. A variant of adeno-associated virus serotype 1 (AAV1) capsid protein,
the variant being a variant of adeno-associated virus serotype 1 (AAV1) capsid protein having one or more mutation selected from D595H, D712E, F713Y, K315R, F584L, A598V and N642H in the amino acid sequence of the wild-type AAV1 capsid protein represented by SEQ ID NO: 1.

2. The variant of the AAV1 capsid protein according to claim 1,
wherein the variant further has one or more mutation selected from T194A, A196S, A197G, T201N, S205A, L129F, A224S, N223S, H272T, S268T, T265S, A263G, S262N and S264T.

3. The variant of the AAV1 capsid protein according to claim 1,
wherein the variant has mutations of D595H, D712E and F713Y.

4. The variant of the AAV1 capsid protein according to claim 1,
wherein the variant has mutations of K315R, F584L, A598V and N642H.

5. The variant of the AAV1 capsid protein according to claim 1,
wherein the variant has mutations of K315R, D595H, D712E and F713Y.

6. The variant of the AAV1 capsid protein according to claim 1,
wherein the variant has mutations of F584L, A598V and N642H.

7. A nucleic acid encoding the variant of the AAV1 capsid protein according to claim 1.

8. The nucleic acid according to claim 6,
which has a base sequence represented by SEQ ID NO: 4.

9. The nucleic acid according to claim 7,
which has a base sequence represented by SEQ ID NO: 6.

10. A recombinant AAV1 vector comprising a nucleic acid encoding the variant of the AAV1 capsid protein according to claim 1.

11. The recombinant AAV1 vector according to claim 10,
wherein the AAV1 vector has an improved transduction profile for astrocytes as compared to an AAV1 wild-type viral vector.

12. The recombinant AAV1 vector according to claim 11,
which is represented by SEQ ID NO: 7.

13. The recombinant AAV1 vector according to claim 12,
which is represented by SEQ ID NO: 8.

14. A pharmaceutical composition comprising the recombinant AAV1 vector according to claim 10.

15. The pharmaceutical composition according to claim 14,
wherein the composition further comprises a pharmaceutically acceptable carrier.

16. The pharmaceutical composition according to claim 14,
which is for preventing or treating a spinal cord injury-related disease.

17. The pharmaceutical composition according to claim 14,
wherein the spinal cord injury-related disease is one selected from a group consisting of spinal cord injury caused by trauma, inflammatory spinal cord injury, vascular spinal cord injury, and tumor-induced spinal cord injury.

18. A gene delivery vehicle comprising the recombinant AAV1 vector according to claim 10.

19. A method for preventing or treating a spinal cord injury-related disease, comprising a step of administering a pharmaceutical composition comprising a therapeutically effective amount of the recombinant AAV1 vector according to claim 10 to a subject.

20. The method for preventing or treating a spinal cord injury-related disease according to claim 19,
wherein the spinal cord injury-related disease is one selected from a group consisting of spinal cord injury caused by trauma, inflammatory spinal cord injury, vascular spinal cord injury, and tumor-induced spinal cord injury.
